# EUROPEAN PATENT APPLICATION

(11) **EP 3 653 708 A1**
(43) Date of publication of application: **20.05.2020**
(21) Application number: 18206235.6
(22) Date of filing: 14.11.2018
(51) Int. Cl.: C12N 9/90, C12P 19/24, C12N 1/21

(54) **ISOMALTULOSE PRODUCTION**

(71) Applicant: Evonik Operations GmbH, 45128 Essen (DE); Technische Universität München - TUM, 80333 München (DE)
(72) Inventor: PILAK, Patrick, 85356 Freising (DE); SKERRA, Arne, 85221 Dachau (DE); ÖHRLEIN, Johannes, 40476 Düsseldorf (DE)
(74) Representative: Evonik Patent Association

(57) **Abstract**

The present invention relates to an isolated sucrose isomerase enzyme (EC 5.4.99.11) comprising at least one point mutation in SEQ ID NO: 1, wherein the point mutation is selected from the group consisting of Q181P, V216A, Y219L, I269V, F270Y, S277Q, S277T, D311P, T369F, D398G, L426V, L426Y, F453Y and combinations thereof.

SEQUENCE LISTING

<110>  Evonik Degussa GmBh

<120>  ISOMALTULOSE PRODUCTION

<130>  2017P00165EP

<160>  115

<170>  PatentIn version 3.5

<210>  1
<211>  573
<212>  PRT
<213>  Serratia plymuthica

<400>  1

Thr Gln Gln Pro Leu Leu Asn Glu Lys Ser Ile Glu Gln Ser Lys Thr
1               5                   10                  15

Ile Pro Lys Trp Trp Lys Glu Ala Val Phe Tyr Gln Val Tyr Pro Arg
            20                  25                  30

Ser Phe Lys Asp Thr Asn Gly Asp Gly Ile Gly Asp Ile Asn Gly Ile
        35                  40                  45

Ile Glu Lys Leu Asp Tyr Leu Lys Ala Leu Gly Ile Asp Ala Ile Trp
    50                  55                  60

Ile Asn Pro His Tyr Asp Ser Pro Asn Thr Asp Asn Gly Tyr Asp Ile
65                  70                  75                  80

Arg Asp Tyr Arg Lys Ile Met Lys Glu Tyr Gly Thr Met Glu Asp Phe
                85                  90                  95

Asp Arg Leu Ile Ser Glu Met Lys Lys Arg Asn Met Arg Leu Met Ile
            100                 105                 110

Asp Val Val Ile Asn His Thr Ser Asp Gln Asn Glu Trp Phe Val Lys
        115                 120                 125

Ser Lys Ser Ser Lys Asp Asn Pro Tyr Arg Gly Tyr Tyr Phe Trp Lys
    130                 135                 140

Asp Ala Lys Glu Gly Gln Ala Pro Asn Asn Tyr Pro Ser Phe Phe Gly
145                 150                 155                 160

Gly Ser Ala Trp Gln Lys Asp Glu Lys Thr Asn Gln Tyr Tyr Leu His
                165                 170                 175

Tyr Phe Ala Lys Gln Gln Pro Asp Leu Asn Trp Asp Asn Pro Lys Val
            180                 185                 190

Arg Gln Asp Leu Tyr Ala Met Leu Arg Phe Trp Leu Asp Lys Gly Val
        195                 200                 205

Ser Gly Leu Arg Phe Asp Thr Val Ala Thr Tyr Ser Lys Ile Pro Asp
    210                 215                 220

Phe Pro Asn Leu Thr Gln Gln Gln Leu Lys Asn Phe Ala Ala Glu Tyr
225                 230                 235                 240

Thr Lys Gly Pro Asn Ile His Arg Tyr Val Asn Glu Met Asn Lys Glu
                245                 250                 255

Val Leu Ser His Tyr Asp Ile Ala Thr Ala Gly Glu Ile Phe Gly Val
            260                 265                 270

Pro Leu Asp Gln Ser Ile Lys Phe Phe Asp Arg Arg Arg Asp Glu Leu
        275                 280                 285

Asn Ile Ala Phe Thr Phe Asp Leu Ile Arg Leu Asp Arg Asp Ser Asp
    290                 295                 300

Gln Arg Trp Arg Arg Lys Asp Trp Lys Leu Ser Gln Phe Arg Gln Ile
305                 310                 315                 320

Ile Asp Asn Val Asp Arg Thr Ala Gly Glu Tyr Gly Trp Asn Ala Phe
                325                 330                 335

Phe Leu Asp Asn His Asp Asn Pro Arg Ala Val Ser His Phe Gly Asp
            340                 345                 350

Asp Arg Pro Gln Trp Arg Glu Pro Ser Ala Lys Ala Leu Ala Thr Leu
        355                 360                 365

Thr Leu Thr Gln Arg Ala Thr Pro Phe Ile Tyr Gln Gly Ser Glu Leu
    370                 375                 380

Gly Met Thr Asn Tyr Pro Phe Lys Ala Ile Asp Glu Phe Asp Asp Ile
385                 390                 395                 400

Glu Val Lys Gly Phe Trp His Asp Tyr Val Glu Thr Gly Lys Val Lys
                405                 410                 415

Ala Asp Glu Phe Leu Gln Asn Val Arg Leu Thr Ser Arg Asp Asn Ser
            420                 425                 430

Arg Thr Pro Phe Gln Trp Asp Gly Ser Lys Asn Ala Gly Phe Thr Ser
        435                 440                 445

Gly Lys Pro Trp Phe Lys Val Asn Pro Asn Tyr Gln Glu Ile Asn Ala
    450                 455                 460

Val Ser Gln Val Thr Gln Pro Asp Ser Val Phe Asn Tyr Tyr Arg Gln
465                 470                 475                 480

Leu Ile Lys Ile Arg His Asp Ile Pro Ala Leu Thr Tyr Gly Thr Tyr
                485                 490                 495

Thr Asp Leu Asp Pro Ala Asn Asp Ser Val Tyr Ala Tyr Thr Arg Ser
            500                 505                 510

Leu Gly Ala Glu Lys Tyr Leu Val Val Val Asn Phe Lys Glu Gln Met
        515                 520                 525

Met Arg Tyr Lys Leu Pro Asp Asn Leu Ser Ile Glu Lys Val Ile Ile
    530                 535                 540

Asp Ser Asn Ser Lys Asn Val Val Lys Lys Asn Asp Ser Leu Leu Glu
545                 550                 555                 560

Leu Lys Pro Trp Gln Ser Gly Val Tyr Lys Leu Asn Gln
                565                 570

<210>  2
<211>  27
<212>  PRT
<213>  Serratia plymuthica

<400>  2

Met Pro Arg Gln Gly Leu Lys Thr Ala Leu Ala Ile Phe Leu Thr Thr
1               5                   10                  15

Ser Leu Cys Ile Ser Cys Gln Gln Ala Phe Gly
            20                  25

<210>  3
<211>  573
<212>  PRT
<213>  Artificial Sequence

<220>
<223>  SmuA + V465E

<400>  3

Thr Gln Gln Pro Leu Leu Asn Glu Lys Ser Ile Glu Gln Ser Lys Thr

1               5                   10                  15

Ile Pro Lys Trp Trp Lys Glu Ala Val Phe Tyr Gln Val Tyr Pro Arg
            20                  25                  30

Ser Phe Lys Asp Thr Asn Gly Asp Gly Ile Gly Asp Ile Asn Gly Ile
        35                  40                  45

Ile Glu Lys Leu Asp Tyr Leu Lys Ala Leu Gly Ile Asp Ala Ile Trp
    50                  55                  60

Ile Asn Pro His Tyr Asp Ser Pro Asn Thr Asp Asn Gly Tyr Asp Ile
65                  70                  75                  80

Arg Asp Tyr Arg Lys Ile Met Lys Glu Tyr Gly Thr Met Glu Asp Phe
                85                  90                  95

Asp Arg Leu Ile Ser Glu Met Lys Lys Arg Asn Met Arg Leu Met Ile
            100                 105                 110

Asp Val Val Ile Asn His Thr Ser Asp Gln Asn Glu Trp Phe Val Lys
        115                 120                 125

Ser Lys Ser Ser Lys Asp Asn Pro Tyr Arg Gly Tyr Tyr Phe Trp Lys
    130                 135                 140

Asp Ala Lys Glu Gly Gln Ala Pro Asn Asn Tyr Pro Ser Phe Phe Gly
145                 150                 155                 160

Gly Ser Ala Trp Gln Lys Asp Glu Lys Thr Asn Gln Tyr Tyr Leu His
                165                 170                 175

Tyr Phe Ala Lys Gln Gln Pro Asp Leu Asn Trp Asp Asn Pro Lys Val
            180                 185                 190

Arg Gln Asp Leu Tyr Ala Met Leu Arg Phe Trp Leu Asp Lys Gly Val
        195                 200                 205

Ser Gly Leu Arg Phe Asp Thr Val Ala Thr Tyr Ser Lys Ile Pro Asp
    210                 215                 220

Phe Pro Asn Leu Thr Gln Gln Gln Leu Lys Asn Phe Ala Ala Glu Tyr
225                 230                 235                 240

Thr Lys Gly Pro Asn Ile His Arg Tyr Val Asn Glu Met Asn Lys Glu
                245                 250                 255

Val Leu Ser His Tyr Asp Ile Ala Thr Ala Gly Glu Ile Phe Gly Val
            260                 265                 270

Pro Leu Asp Gln Ser Ile Lys Phe Phe Asp Arg Arg Arg Asp Glu Leu
        275                 280                 285

Asn Ile Ala Phe Thr Phe Asp Leu Ile Arg Leu Asp Arg Asp Ser Asp
    290                 295                 300

Gln Arg Trp Arg Arg Lys Asp Trp Lys Leu Ser Gln Phe Arg Gln Ile
305                 310                 315                 320

Ile Asp Asn Val Asp Arg Thr Ala Gly Glu Tyr Gly Trp Asn Ala Phe
                325                 330                 335

Phe Leu Asp Asn His Asp Asn Pro Arg Ala Val Ser His Phe Gly Asp
            340                 345                 350

Asp Arg Pro Gln Trp Arg Glu Pro Ser Ala Lys Ala Leu Ala Thr Leu
        355                 360                 365

Thr Leu Thr Gln Arg Ala Thr Pro Phe Ile Tyr Gln Gly Ser Glu Leu
    370                 375                 380

Gly Met Thr Asn Tyr Pro Phe Lys Ala Ile Asp Glu Phe Asp Asp Ile
385                 390                 395                 400

Glu Val Lys Gly Phe Trp His Asp Tyr Val Glu Thr Gly Lys Val Lys
                405                 410                 415

Ala Asp Glu Phe Leu Gln Asn Val Arg Leu Thr Ser Arg Asp Asn Ser
            420                 425                 430

Arg Thr Pro Phe Gln Trp Asp Gly Ser Lys Asn Ala Gly Phe Thr Ser
        435                 440                 445

Gly Lys Pro Trp Phe Lys Val Asn Pro Asn Tyr Gln Glu Ile Asn Ala
    450                 455                 460

Glu Ser Gln Val Thr Gln Pro Asp Ser Val Phe Asn Tyr Tyr Arg Gln
465                 470                 475                 480

Leu Ile Lys Ile Arg His Asp Ile Pro Ala Leu Thr Tyr Gly Thr Tyr
                485                 490                 495

Thr Asp Leu Asp Pro Ala Asn Asp Ser Val Tyr Ala Tyr Thr Arg Ser
            500                 505                 510

Leu Gly Ala Glu Lys Tyr Leu Val Val Val Asn Phe Lys Glu Gln Met
        515                 520                 525

Met Arg Tyr Lys Leu Pro Asp Asn Leu Ser Ile Glu Lys Val Ile Ile
    530                 535                 540

Asp Ser Asn Ser Lys Asn Val Val Lys Lys Asn Asp Ser Leu Leu Glu
545                 550                 555                 560

Leu Lys Pro Trp Gln Ser Gly Val Tyr Lys Leu Asn Gln
                565                 570

<210>  4
<211>  4808
<212>  DNA
<213>  Artificial Sequence

<220>
<223>  pASK-IBA4(+)-SmuA

<400>  4
tagagaaaag tgaaatgaat agttcgacaa aaatctagat aacgagggca aaaaatgaaa      60

aagacagcta tcgcgattgc agtggcactg gctggtttcg ctaccgtagc gcaggccgct     120

agctggagcc acccgcagtt cgaaaaaggc gccaccatcc cgaaatggtg gaaagaagct     180

gttttctacc aggtttaccc gcgttccttc aaagacacca acggtgacgg tatcggtgac     240

atcaacggta tcatcgaaaa actggactac ctgaaagctc tgggtatcga cgctatctgg     300

atcaacccgc actacgactc cccgaacacc gacaacggtt acgacatccg tgactaccgt     360

aaaatcatga aagaatacgg taccatggaa gacttcgacc gtctgatctc cgaaatgaaa     420

aaacgtaaca tgcgtctgat gatcgacgtt gttatcaacc acacctccga ccagaacgaa     480

tggttcgtta aatccaaatc ctccaaagac aacccgtacc gtggttacta cttctggaaa     540

gacgctaaag aaggtcaggc tccgaacaac tacccgtcct tcttcggtgg ttccgcttgg     600

cagaaagacg aaaaaaccaa ccagtactac ctgcactact tcgctaaaca gcagccggac     660

ctgaactggg acaacccgaa agttcgtcag gacctgtacg ctatgctgcg tttctggctg     720

gacaaaggtg tttccggtct gcgtttcgac accgttgcta cctactccaa aatcccggac     780

ttcccgaacc tgacccagca gcagctgaaa aacttcgctg ctgaatacac caaaggtccg     840

aacatccacc gttacgttaa cgaaatgaac aaagaagttc tgtcccacta cgacatcgct     900

accgctggtg aaatcttcgg tgttccgctg gaccagtcca tcaaattctt cgaccgtcgt     960

cgtgacgaac tgaacatcgc tttcaccttc gacctgatcc gtctggaccg tgactccgac    1020

cagcgttggc gtcgtaaaga ctggaaactg tcccagttcc gtcagatcat cgacaacgtt    1080

gaccgtaccg ctggtgaata cggttggaac gctttcttcc tggacaacca cgacaacccg    1140

cgtgctgttt cccacttcgg tgacgaccgt ccgcagtggc gtgaaccgtc cgctaaagct 1200

ctggctaccc tgaccctgac ccagcgtgct accccgttca tctaccaggg ttccgaactg 1260

ggtatgacca actacccgtt caaagctatc gacgaattcg acgacatcga agttaaaggt 1320

ttctggcacg actacgttga aaccggtaaa gttaaagctg acgaattcct gcagaacgtt 1380

cgtctgacct cccgtgacaa ctcccgtacc ccgttccagt gggacggttc caaaaacgct 1440

ggtttcacct ccggtaaacc gtggttcaaa gttaacccga actaccagga aatcaacgct 1500

gaatcccagg ttacccagcc ggactccgtt ttcaactact accgtcagct gatcaaaatc 1560

cgtcacgaca tcccggctct gacctacggt acctacaccg acctggaccc ggctaacgac 1620

tccgtttacg cttacacccg ttccctgggt gctgaaaaat acctggttgt tgttaacttc 1680

aaagaacaga tgatgcgtta caaactgccg gacaacctgt ccatcgaaaa agttatcatc 1740

gactccaact ccaaaaacgt tgttaaaaaa aacgactccc tgctggaact gaaaccgtgg 1800

cagtccggtg tttacaaact gaaccagtaa gcttgacctg tgaagtgaaa aatggcgcac 1860

attgtgcgac attttttttg tctgccgttt accgctactg cgtcacggat ctccacgcgc 1920

cctgtagcgg cgcattaagc gcggcgggtg tggtggttac gcgcagcgtg accgctacac 1980

ttgccagcgc cctagcgccc gctcctttcg ctttcttccc ttcctttctc gccacgttcg 2040

ccggctttcc ccgtcaagct ctaaatcggg ggctcccttt agggttccga tttagtgctt 2100

tacggcacct cgaccccaaa aaacttgatt agggtgatgg ttcacgtagt gggccatcgc 2160

cctgatagac ggtttttcgc cctttgacgt tggagtccac gttctttaat agtggactct 2220

tgttccaaac tggaacaaca ctcaacccta tctcggtcta ttcttttgat ttataaggga 2280

ttttgccgat ttcggcctat tggttaaaaa atgagctgat ttaacaaaaa tttaacgcga 2340

attttaacaa aatattaacg cttacaattt caggtggcac ttttcgggga aatgtgcgcg 2400

gaacccctat ttgtttattt ttctaaatac attcaaatat gtatccgctc atgagacaat 2460

aaccctgata aatgcttcaa taatattgaa aaaggaagag tatgagtatt caacatttcc 2520

gtgtcgccct tattcccttt tttgcggcat tttgccttcc tgtttttgct cacccagaaa 2580

cgctggtgaa agtaaaagat gctgaagatc agttgggtgc acgagtgggt tacatcgaac 2640

tggatctcaa cagcggtaag atccttgaga gttttcgccc cgaagaacgt tttccaatga 2700

tgagcacttt taaagttctg ctatgtggcg cggtattatc ccgtattgac gccgggcaag 2760

agcaactcgg tcgccgcata cactattctc agaatgactt ggttgagtac tcaccagtca 2820

cagaaaagca tcttacggat ggcatgacag taagagaatt atgcagtgct gccataacca 2880

tgagtgataa cactgcggcc aacttacttc tgacaacgat cggaggaccg aaggagctaa 2940

ccgctttttt gcacaacatg ggggatcatg taactcgcct tgatcgttgg gaaccggagc 3000

tgaatgaagc cataccaaac gacgagcgtg acaccacgat gcctgtagca atggcaacaa 3060

cgttgcgcaa actattaact ggcgaactac ttactctagc ttcccggcaa caattgatag 3120

actggatgga ggcggataaa gttgcaggac cacttctgcg ctcggccctt ccggctggct 3180

ggtttattgc tgataaatct ggagccggtg agcgtggctc tcgcggtatc attgcagcac 3240

tggggccaga tggtaagccc tcccgtatcg tagttatcta cacgacgggg agtcaggcaa 3300

ctatggatga acgaaataga cagatcgctg agataggtgc ctcactgatt aagcattggt 3360

aggaattaat gatgtctcgt ttagataaaa gtaaagtgat taacagcgca ttagagctgc 3420

ttaatgaggt cggaatcgaa ggtttaacaa cccgtaaact cgcccagaag ctaggtgtag 3480

agcagcctac attgtattgg catgtaaaaa ataagcgggc tttgctcgac gccttagcca 3540

ttgagatgtt agataggcac catactcact tttgcccttt agaaggggaa agctggcaag 3600

attttttacg taataacgct aaaagtttta gatgtgcttt actaagtcat cgcgatggag 3660

caaaagtaca tttaggtaca cggcctacag aaaaacagta tgaaactctc gaaaatcaat 3720

tagccttttt atgccaacaa ggtttttcac tagagaatgc attatatgca ctcagcgcag 3780

tggggcattt tactttaggt tgcgtattgg aagatcaaga gcatcaagtc gctaaagaag 3840

aaagggaaac acctactact gatagtatgc cgccattatt acgacaagct atcgaattat 3900

ttgatcacca aggtgcagag ccagccttct tattcggcct tgaattgatc atatgcggat 3960

tagaaaaaca acttaaatgt gaaagtgggt cttaaaagca gcataacctt tttccgtgat 4020

ggtaacttca ctagtttaaa aggatctagg tgaagatcct ttttgataat ctcatgacca 4080

aaatccctta acgtgagttt tcgttccact gagcgtcaga ccccgtagaa aagatcaaag 4140

gatcttcttg agatcctttt tttctgcgcg taatctgctg cttgcaaaca aaaaaaccac 4200

cgctaccagc ggtggtttgt ttgccggatc aagagctacc aactcttttt ccgaaggtaa 4260

ctggcttcag cagagcgcag ataccaaata ctgtccttct agtgtagccg tagttaggcc 4320

accacttcaa gaactctgta gcaccgccta catacctcgc tctgctaatc ctgttaccag 4380

tggctgctgc cagtggcgat aagtcgtgtc ttaccgggtt ggactcaaga cgatagttac 4440

cggataaggc gcagcggtcg ggctgaacgg ggggttcgtg cacacagccc agcttggagc 4500

gaacgaccta caccgaactg agatacctac agcgtgagct atgagaaagc gccacgcttc 4560

ccgaagggag aaaggcggac aggtatccgg taagcggcag ggtcggaaca ggagagcgca 4620

cgagggagct tccaggggga aacgcctggt atctttatag tcctgtcggg tttcgccacc 4680

tctgacttga gcgtcgattt ttgtgatgct cgtcaggggg gcggagccta tggaaaaacg 4740

ccagcaacgc ggccttttta cggttcctgg ccttttgctg gccttttgct cacatgaccc 4800

gacaccat 4808

<210>  5
<211>  558
<212>  PRT
<213>  Serratia plymuthica

<400>  5

Thr Ile Pro Lys Trp Trp Lys Glu Ala Val Phe Tyr Gln Val Tyr Pro
1               5                   10                  15

Arg Ser Phe Lys Asp Thr Asn Gly Asp Gly Ile Gly Asp Ile Asn Gly
            20                  25                  30

Ile Ile Glu Lys Leu Asp Tyr Leu Lys Ala Leu Gly Ile Asp Ala Ile
        35                  40                  45

Trp Ile Asn Pro His Tyr Asp Ser Pro Asn Thr Asp Asn Gly Tyr Asp
    50                  55                  60

Ile Arg Asp Tyr Arg Lys Ile Met Lys Glu Tyr Gly Thr Met Glu Asp
65                  70                  75                  80

Phe Asp Arg Leu Ile Ser Glu Met Lys Lys Arg Asn Met Arg Leu Met
                85                  90                  95

Ile Asp Val Val Ile Asn His Thr Ser Asp Gln Asn Glu Trp Phe Val
            100                 105                 110

Lys Ser Lys Ser Ser Lys Asp Asn Pro Tyr Arg Gly Tyr Tyr Phe Trp
        115                 120                 125

Lys Asp Ala Lys Glu Gly Gln Ala Pro Asn Asn Tyr Pro Ser Phe Phe
    130                 135                 140

Gly Gly Ser Ala Trp Gln Lys Asp Glu Lys Thr Asn Gln Tyr Tyr Leu
145                 150                 155                 160

His Tyr Phe Ala Lys Gln Gln Pro Asp Leu Asn Trp Asp Asn Pro Lys
                165                 170                 175

Val Arg Gln Asp Leu Tyr Ala Met Leu Arg Phe Trp Leu Asp Lys Gly
            180                 185                 190

Val Ser Gly Leu Arg Phe Asp Thr Val Ala Thr Tyr Ser Lys Ile Pro
        195                 200                 205

Asp Phe Pro Asn Leu Thr Gln Gln Gln Leu Lys Asn Phe Ala Ala Glu
    210                 215                 220

Tyr Thr Lys Gly Pro Asn Ile His Arg Tyr Val Asn Glu Met Asn Lys
225                 230                 235                 240

Glu Val Leu Ser His Tyr Asp Ile Ala Thr Ala Gly Glu Ile Phe Gly
                245                 250                 255

Val Pro Leu Asp Gln Ser Ile Lys Phe Phe Asp Arg Arg Arg Asp Glu
            260                 265                 270

Leu Asn Ile Ala Phe Thr Phe Asp Leu Ile Arg Leu Asp Arg Asp Ser
        275                 280                 285

Asp Gln Arg Trp Arg Arg Lys Asp Trp Lys Leu Ser Gln Phe Arg Gln
    290                 295                 300

Ile Ile Asp Asn Val Asp Arg Thr Ala Gly Glu Tyr Gly Trp Asn Ala
305                 310                 315                 320

Phe Phe Leu Asp Asn His Asp Asn Pro Arg Ala Val Ser His Phe Gly
                325                 330                 335

Asp Asp Arg Pro Gln Trp Arg Glu Pro Ser Ala Lys Ala Leu Ala Thr
            340                 345                 350

Leu Thr Leu Thr Gln Arg Ala Thr Pro Phe Ile Tyr Gln Gly Ser Glu
        355                 360                 365

Leu Gly Met Thr Asn Tyr Pro Phe Lys Ala Ile Asp Glu Phe Asp Asp
    370                 375                 380

Ile Glu Val Lys Gly Phe Trp His Asp Tyr Val Glu Thr Gly Lys Val
385                 390                 395                 400

Lys Ala Asp Glu Phe Leu Gln Asn Val Arg Leu Thr Ser Arg Asp Asn
                405                 410                 415

Ser Arg Thr Pro Phe Gln Trp Asp Gly Ser Lys Asn Ala Gly Phe Thr
            420                 425                 430

Ser Gly Lys Pro Trp Phe Lys Val Asn Pro Asn Tyr Gln Glu Ile Asn
        435                 440                 445

Ala Glu Ser Gln Val Thr Gln Pro Asp Ser Val Phe Asn Tyr Tyr Arg
    450                 455                 460

Gln Leu Ile Lys Ile Arg His Asp Ile Pro Ala Leu Thr Tyr Gly Thr
465                 470                 475                 480

Tyr Thr Asp Leu Asp Pro Ala Asn Asp Ser Val Tyr Ala Tyr Thr Arg
                485                 490                 495

Ser Leu Gly Ala Glu Lys Tyr Leu Val Val Val Asn Phe Lys Glu Gln
            500                 505                 510

Met Met Arg Tyr Lys Leu Pro Asp Asn Leu Ser Ile Glu Lys Val Ile
        515                 520                 525

Ile Asp Ser Asn Ser Lys Asn Val Val Lys Lys Asn Asp Ser Leu Leu
    530                 535                 540

Glu Leu Lys Pro Trp Gln Ser Gly Val Tyr Lys Leu Asn Gln
545                 550                 555

<210>  6
<211>  27
<212>  DNA
<213>  Artificial Sequence

<220>
<223>  Oligonucleotide SmuA_P67S_For

<400>  6
atctggatca actcgcacta cgactcc                                          27

<210>  7
<211>  27
<212>  DNA
<213>  Artificial Sequence

<220>
<223>  Oligonucleotide SmuA_P67S_Rev

<400>  7
ggagtcgtag tgcgagttga tccagat                                          27

<210>  8
<211>  27
<212>  DNA
<213>  Artificial Sequence

<220>
<223>  Oligonucleotide SmuA_Y78W_For

<400>  8
accgacaacg gttgggacat ccgtgac                                          27

<210>  9
<211>  27
<212>  DNA
<213>  Artificial Sequence

<220>

<223>  Oligonucleotide SmuA_Y78W_Rev

<400>  9
gtcacggatg tcccaaccgt tgtcggt                                          27


<210>  10
<211>  27
<212>  DNA
<213>  Artificial Sequence

<220>
<223>  Oligonucleotide SmuA_A179V_For

<400>  10
ctgcactact tcgttaaaca gcagccg                                          27


<210>  11
<211>  27
<212>  DNA
<213>  Artificial Sequence

<220>
<223>  Oligonucleotide SmuA_A179V_Rev

<400>  11
cggctgctgt ttaacgaagt agtgcag                                          27


<210>  12
<211>  29
<212>  DNA
<213>  Artificial Sequence

<220>
<223>  Oligonucleotide SmuA_K180G_For

<400>  12
gcactacttc gctggacagc agccggacc                                        29


<210>  13
<211>  29
<212>  DNA
<213>  Artificial Sequence

<220>
<223>  Oligonucleotide SmuA_K180G_Rev

<400>  13
ggtccggctg ctgtccagcg aagtagtgc                                        29


<210>  14
<211>  29
<212>  DNA
<213>  Artificial Sequence

<220>
<223>  Oligonucleotide SmuA_K180V_For

<400>  14

gcactacttc gctgtacagc agccggacc                                    29

<210>  15
<211>  29
<212>  DNA
<213>  Artificial Sequence

<220>
<223>  Oligonucleotide SmuA_K180V_Rev

<400>  15
ggtccggctg ctgtacagcg aagtagtgc                                    29

<210>  16
<211>  27
<212>  DNA
<213>  Artificial Sequence

<220>
<223>  Oligonucleotide SmuA_Q181P_For

<400>  16
tacttcgcta aaccgcagcc ggacctg                                      27

<210>  17
<211>  27
<212>  DNA
<213>  Artificial Sequence

<220>
<223>  Oligonucleotide SmuA_Q181P_Rev

<400>  17
caggtccggc tgcggtttag cgaagta                                      27

<210>  18
<211>  27
<212>  DNA
<213>  Artificial Sequence

<220>
<223>  Oligonucleotide SmuA_F213L_For

<400>  18
ttccggtctg cgtctcgaca ccgttgc                                      27

<210>  19
<211>  27
<212>  DNA
<213>  Artificial Sequence

<220>
<223>  Oligonucleotide SmuA_F213L_Rev

<400>  19
gcaacggtgt cgagacgcag accggaa                                      27

<210>  20
<211>  27
<212>  DNA
<213>  Artificial Sequence

<220>
<223>  Oligonucleotide SmuA_V216A_For

<400>  20
cgtttcgaca ccgctgctac ctactcc                                          27


<210>  21
<211>  27
<212>  DNA
<213>  Artificial Sequence

<220>
<223>  Oligonucleotide SmuA_V216A_Rev

<400>  21
ggagtaggta gcagcggtgt cgaaacg                                          27


<210>  22
<211>  26
<212>  DNA
<213>  Artificial Sequence

<220>
<223>  Oligonucleotide SmuA_A217V_For

<400>  22
gtttcgacac cgttgttacc tactcc                                           26


<210>  23
<211>  26
<212>  DNA
<213>  Artificial Sequence

<220>
<223>  Oligonucleotide SmuA_A217V_Rev

<400>  23
ggagtaggta acaacggtgt cgaaac                                           26


<210>  24
<211>  27
<212>  DNA
<213>  Artificial Sequence

<220>
<223>  Oligonucleotide SmuA_Y219L_For

<400>  24
accgttgcta ccttgtccaa aatcccg                                          27


<210>  25
<211>  27
<212>  DNA

<213>  Artificial Sequence

<220>
<223>  Oligonucleotide SmuA_Y219L_Rev

<400>  25
   cgggattttg gacaaggtag caacggt                                          27


<210>  26
<211>  30
<212>  DNA
<213>  Artificial Sequence

<220>
<223>  Oligonucleotide SmuA_F236Y_For

<400>  26
gcagctgaaa aactacgctg ctgaatacac                                      30


<210>  27
<211>  30
<212>  DNA
<213>  Artificial Sequence

<220>
<223>  Oligonucleotide SmuA_F236Y_Rev

<400>  27
gtgtattcag cagcgtagtt tttcagctgc                                      30


<210>  28
<211>  30
<212>  DNA
<213>  Artificial Sequence

<220>
<223>  Oligonucleotide SmuA_I269A_For

<400>  28
ctaccgctgg tgaagccttc ggtgttccgc                                      30


<210>  29
<211>  30
<212>  DNA
<213>  Artificial Sequence

<220>
<223>  Oligonucleotide SmuA_I269A_Rev

<400>  29
gcggaacacc gaaggcttca ccagcggtag                                      30


<210>  30
<211>  30
<212>  DNA
<213>  Artificial Sequence

<220>

<223>  Oligonucleotide SmuA_I269L_For

<400>  30
ctaccgctgg tgaactcttc ggtgttccgc                                        30


<210>  31
<211>  30
<212>  DNA
<213>  Artificial Sequence

<220>
<223>  Oligonucleotide SmuA_I269L_Rev

<400>  31
gcggaacacc gaagagttca ccagcggtag                                        30


<210>  32
<211>  30
<212>  DNA
<213>  Artificial Sequence

<220>
<223>  Oligonucleotide SmuA_I269V_For

<400>  32
ctaccgctgg tgaagtcttc ggtgttccgc                                        30


<210>  33
<211>  30
<212>  DNA
<213>  Artificial Sequence

<220>
<223>  Oligonucleotide SmuA_I269V_Rev

<400>  33
gcggaacacc gaagacttca ccagcggtag                                        30


<210>  34
<211>  27
<212>  DNA
<213>  Artificial Sequence

<220>
<223>  Oligonucleotide SmuA_F270Y_For

<400>  34
gctggtgaaa tctacggtgt tccgctg                                           27


<210>  35
<211>  27
<212>  DNA
<213>  Artificial Sequence

<220>
<223>  Oligonucleotide SmuA_F270Y_Rev

<400>  35

cagcggaaca ccgtagattt caccagc                                          27

<210>  36
<211>  28
<212>  DNA
<213>  Artificial Sequence

<220>
<223>  Oligonucleotide SmuA_S277M_For

<400>  36
ccgctggacc agatgatcaa attcttcg                                         28

<210>  37
<211>  28
<212>  DNA
<213>  Artificial Sequence

<220>
<223>  Oligonucleotide SmuA_S277M_Rev

<400>  37
cgaagaattt gatcatctgg tccagcgg                                         28

<210>  38
<211>  28
<212>  DNA
<213>  Artificial Sequence

<220>
<223>  Oligonucleotide SmuA_S277Q_For

<400>  38
ccgctggacc agcagatcaa attcttcg                                         28

<210>  39
<211>  28
<212>  DNA
<213>  Artificial Sequence

<220>
<223>  Oligonucleotide SmuA_S277Q_Rev

<400>  39
cgaagaattt gatctgctgg tccagcgg                                         28

<210>  40
<211>  28
<212>  DNA
<213>  Artificial Sequence

<220>
<223>  Oligonucleotide SmuA_S277T_For

<400>  40
ccgctggacc agaccatcaa attcttcg                                         28

<210>  41
<211>  28
<212>  DNA
<213>  Artificial Sequence

<220>
<223>  Oligonucleotide SmuA_S277T_Rev

<400>  41
cgaagaattt gatggtctgg tccagcgg                                          28


<210>  42
<211>  27
<212>  DNA
<213>  Artificial Sequence

<220>
<223>  Oligonucleotide SmuA_R284P_For

<400>  42
ttcttcgacc gtccgcgtga cgaactg                                           27


<210>  43
<211>  27
<212>  DNA
<213>  Artificial Sequence

<220>
<223>  Oligonucleotide SmuA_R284P_Rev

<400>  43
cagttcgtca cgcggacggt cgaagaa                                           27


<210>  44
<211>  27
<212>  DNA
<213>  Artificial Sequence

<220>
<223>  Oligonucleotide SmuA_F294Y_For

<400>  44
tcgctttcac ctacgacctg atccgtc                                           27


<210>  45
<211>  27
<212>  DNA
<213>  Artificial Sequence

<220>
<223>  Oligonucleotide SmuA_F294Y_Rev

<400>  45
gacggatcag gtcgtaggtg aaagcga                                           27


<210>  46
<211>  27
<212>  DNA

<213>  Artificial Sequence

<220>
<223>  Oligonucleotide SmuA_I297G_For

<400>  46
ttcgacctgg gccgtctgga ccgtgac                                          27


<210>  47
<211>  27
<212>  DNA
<213>  Artificial Sequence

<220>
<223>  Oligonucleotide SmuA_I297G_Rev

<400>  47
gtcacggtcc agacggccca ggtcgaa                                          27


<210>  48
<211>  27
<212>  DNA
<213>  Artificial Sequence

<220>
<223>  Oligonucleotide SmuA_L299G_For

<400>  48
acctgatccg tggggaccgt gactccg                                          27


<210>  49
<211>  27
<212>  DNA
<213>  Artificial Sequence

<220>
<223>  Oligonucleotide SmuA_L299G_Rev

<400>  49
cggagtcacg gtccccacgg atcaggt                                          27


<210>  50
<211>  30
<212>  DNA
<213>  Artificial Sequence

<220>
<223>  Oligonucleotide SmuA_D311P_For

<400>  50
gttggcgtcg taaaccgtgg aaactgtccc                                       30


<210>  51
<211>  30
<212>  DNA
<213>  Artificial Sequence

<220>

<223>  Oligonucleotide SmuA_D311P_Rev

<400>  51
gggacagttt ccacggttta cgacgccaac                                        30


<210>  52
<211>  27
<212>  DNA
<213>  Artificial Sequence

<220>
<223>  Oligonucleotide SmuA_H341F_For

<400>  52
tcctggacaa cttcgacaac ccgcgtg                                           27


<210>  53
<211>  27
<212>  DNA
<213>  Artificial Sequence

<220>
<223>  Oligonucleotide SmuA_H341F_Rev

<400>  53
cacgcgggtt gtcgaagttg tccagga                                           27


<210>  54
<211>  27
<212>  DNA
<213>  Artificial Sequence

<220>
<223>  Oligonucleotide SmuA_T369F_For

<400>  54
tggctaccct gttcctgacc cagcgtg                                           27


<210>  55
<211>  27
<212>  DNA
<213>  Artificial Sequence

<220>
<223>  Oligonucleotide SmuA_T369F_Rev

<400>  55
cacgctgggt caggaacagg gtagcca                                           27


<210>  56
<211>  27
<212>  DNA
<213>  Artificial Sequence

<220>
<223>  Oligonucleotide SmuA_T369W_For

<400>  56

tggctaccct gtggctgacc cagcgtg                                          27

<210>  57
<211>  27
<212>  DNA
<213>  Artificial Sequence

<220>
<223>  Oligonucleotide SmuA_T369W_Rev

<400>  57
cacgctgggt cagccacagg gtagcca                                          27

<210>  58
<211>  27
<212>  DNA
<213>  Artificial Sequence

<220>
<223>  Oligonucleotide SmuA_D398G_For

<400>  58
ctatcgacga attcggcgac atcgaag                                          27

<210>  59
<211>  27
<212>  DNA
<213>  Artificial Sequence

<220>
<223>  Oligonucleotide SmuA_D398G_Rev

<400>  59
cttcgatgtc gccgaattcg tcgatag                                          27

<210>  60
<211>  27
<212>  DNA
<213>  Artificial Sequence

<220>
<223>  Oligonucleotide SmuA_D398P_For

<400>  60
ctatcgacga attcccggac atcgaag                                          27

<210>  61
<211>  27
<212>  DNA
<213>  Artificial Sequence

<220>
<223>  Oligonucleotide SmuA_D398P_Rev

<400>  61
cttcgatgtc cgggaattcg tcgatag                                          27

<210>  62
<211>  27
<212>  DNA
<213>  Artificial Sequence

<220>
<223>  Oligonucleotide SmuA_D399E_For

<400>  62
acgaattcga cgaaatcgag gttaaag                                          27


<210>  63
<211>  27
<212>  DNA
<213>  Artificial Sequence

<220>
<223>  Oligonucleotide SmuA_D399E_Rev

<400>  63
ctttaacctc gatttcgtcg aattcgt                                          27


<210>  64
<211>  30
<212>  DNA
<213>  Artificial Sequence

<220>
<223>  Oligonucleotide SmuA_D399N_For

<400>  64
cgacgaattc gacaacatcg aagttaaagg                                       30


<210>  65
<211>  30
<212>  DNA
<213>  Artificial Sequence

<220>
<223>  Oligonucleotide SmuA_D399N_Rev

<400>  65
cctttaactt cgatgttgtc gaattcgtcg                                       30


<210>  66
<211>  27
<212>  DNA
<213>  Artificial Sequence

<220>
<223>  Oligonucleotide SmuA_D399S_For

<400>  66
acgaattcga cagcatcgag gttaaag                                          27


<210>  67
<211>  27
<212>  DNA

<213>  Artificial Sequence

<220>
<223>  Oligonucleotide SmuA_D399S_Rev

<400>  67
ctttaacctc gatgctgtcg aattcgt                                          27


<210>  68
<211>  29
<212>  DNA
<213>  Artificial Sequence

<220>
<223>  Oligonucleotide SmuA_I400A_For

<400>  68
gacgaattcg acgacgccga agttaaagg                                        29


<210>  69
<211>  29
<212>  DNA
<213>  Artificial Sequence

<220>
<223>  Oligonucleotide SmuA_I400A_Rev

<400>  69
cctttaactt cggcgtcgtc gaattcgtc                                        29


<210>  70
<211>  29
<212>  DNA
<213>  Artificial Sequence

<220>
<223>  Oligonucleotide SmuA_I400V_For

<400>  70
gacgaattcg acgacgtcga agttaaagg                                        29


<210>  71
<211>  29
<212>  DNA
<213>  Artificial Sequence

<220>
<223>  Oligonucleotide SmuA_I400V_Rev

<400>  71
cctttaactt cgacgtcgtc gaattcgtc                                        29


<210>  72
<211>  27
<212>  DNA
<213>  Artificial Sequence

<220>

<223>  Oligonucleotide SmuA_I400Y_For

<400>  72
cgaattcgac gactatgaag ttaaagg                                          27


<210>  73
<211>  27
<212>  DNA
<213>  Artificial Sequence

<220>
<223>  Oligonucleotide SmuA_I400Y_Rev

<400>  73
cctttaactt catagtcgtc gaattcg                                          27


<210>  74
<211>  27
<212>  DNA
<213>  Artificial Sequence

<220>
<223>  Oligonucleotide SmuA_E401D_For

<400>  74
ttcgacgaca tcgatgttaa aggtttc                                          27


<210>  75
<211>  27
<212>  DNA
<213>  Artificial Sequence

<220>
<223>  Oligonucleotide SmuA_E401D_Rev

<400>  75
gaaaccttta acatcgatgt cgtcgaa                                          27


<210>  76
<211>  27
<212>  DNA
<213>  Artificial Sequence

<220>
<223>  Oligonucleotide SmuA_E401Q_For

<400>  76
ttcgacgaca tccaagttaa aggtttc                                          27


<210>  77
<211>  27
<212>  DNA
<213>  Artificial Sequence

<220>
<223>  Oligonucleotide SmuA_E401Q_Rev

<400>  77

gaaaccttta acttggatgt cgtcgaa                                          27

<210>  78
<211>  27
<212>  DNA
<213>  Artificial Sequence

<220>
<223>  Oligonucleotide SmuA_L426V_For

<400>  78
cagaacgttc gtgtgacctc ccgtgac                                          27

<210>  79
<211>  27
<212>  DNA
<213>  Artificial Sequence

<220>
<223>  Oligonucleotide SmuA_L426V_Rev

<400>  79
gtcacgggag gtcacacgaa cgttctg                                          27

<210>  80
<211>  27
<212>  DNA
<213>  Artificial Sequence

<220>
<223>  Oligonucleotide SmuA_L426Y_For

<400>  80
cagaacgttc gttacacctc ccgtgac                                          27

<210>  81
<211>  27
<212>  DNA
<213>  Artificial Sequence

<220>
<223>  Oligonucleotide SmuA_L426Y_Rev

<400>  81
gtcacgggag gtgtaacgaa cgttctg                                          27

<210>  82
<211>  28
<212>  DNA
<213>  Artificial Sequence

<220>
<223>  Oligonucleotide SmuA_T427I_For

<400>  82
gaacgttcgt ctgatctccg tgacaact                                         28

<210>  83
<211>  29
<212>  DNA
<213>  Artificial Sequence

<220>
<223>  Oligonucleotide SmuA_T427I_Rev

<400>  83
agttgtcacg ggagatcaga cgaacgttc                                          29


<210>  84
<211>  29
<212>  DNA
<213>  Artificial Sequence

<220>
<223>  Oligonucleotide SmuA_T427S_For

<400>  84
gaacgttcgt ctgagctccc gtgacaact                                          29


<210>  85
<211>  29
<212>  DNA
<213>  Artificial Sequence

<220>
<223>  Oligonucleotide SmuA_T427S_Rev

<400>  85
agttgtcacg ggagctcaga cgaacgttc                                          29


<210>  86
<211>  29
<212>  DNA
<213>  Artificial Sequence

<220>
<223>  Oligonucleotide SmuA_T427V_For

<400>  86
gaacgttcgt ctggtctccc gtgacaact                                          29


<210>  87
<211>  29
<212>  DNA
<213>  Artificial Sequence

<220>
<223>  Oligonucleotide SmuA_T427V_Rev

<400>  87
agttgtcacg ggagaccaga cgaacgttc                                          29


<210>  88
<211>  30
<212>  DNA

<213>  Artificial Sequence

<220>
<223>  Oligonucleotide SmuA_S428A_For

<400>  88
cgttcgtctg accgcccgtg acaactcccg                                        30


<210>  89
<211>  30
<212>  DNA
<213>  Artificial Sequence

<220>
<223>  Oligonucleotide SmuA_S428A_Rev

<400>  89
cgggagttgt cacgggcggt cagacgaacg                                        30


<210>  90
<211>  30
<212>  DNA
<213>  Artificial Sequence

<220>
<223>  Oligonucleotide SmuA_S428T_For

<400>  90
cgttcgtctg accacccgtg acaactcccg                                        30


<210>  91
<211>  30
<212>  DNA
<213>  Artificial Sequence

<220>
<223>  Oligonucleotide SmuA_S428T_Rev

<400>  91
cgggagttgt cacgggtggt cagacgaacg                                        30


<210>  92
<211>  29
<212>  DNA
<213>  Artificial Sequence

<220>
<223>  Oligonucleotide SmuA_D430A_For

<400>  92
gtctgacctc ccgtgccaac tcccgtacc                                         29


<210>  93
<211>  29
<212>  DNA
<213>  Artificial Sequence

<220>

<223>  Oligonucleotide SmuA_D430A_Rev

<400>  93
ggtacgggag ttggcacggg aggtcagac                                          29


<210>  94
<211>  29
<212>  DNA
<213>  Artificial Sequence

<220>
<223>  Oligonucleotide SmuA_D430E_For

<400>  94
gtctgacctc ccgtgagaac tcccgtacc                                          29


<210>  95
<211>  29
<212>  DNA
<213>  Artificial Sequence

<220>
<223>  Oligonucleotide SmuA_D430E_Rev

<400>  95
ggtacgggag ttctcacggg aggtcagac                                          29


<210>  96
<211>  29
<212>  DNA
<213>  Artificial Sequence

<220>
<223>  Oligonucleotide SmuA_D430L_For

<400>  96
gtctgacctc ccgtctgaac tcccgtacc                                          29


<210>  97
<211>  29
<212>  DNA
<213>  Artificial Sequence

<220>
<223>  Oligonucleotide SmuA_D430L_Rev

<400>  97
ggtacgggag ttcagacggg aggtcagac                                          29


<210>  98
<211>  29
<212>  DNA
<213>  Artificial Sequence

<220>
<223>  Oligonucleotide SmuA_D430N_For

<400>  98

gtctgacctc ccgtaacaac tcccgtacc                                          29

<210>  99
<211>  29
<212>  DNA
<213>  Artificial Sequence

<220>
<223>  Oligonucleotide SmuA_D430N_Rev

<400>  99
ggtacgggag ttgttacggg aggtcagac                                          29

<210>  100
<211>  29
<212>  DNA
<213>  Artificial Sequence

<220>
<223>  Oligonucleotide SmuA_D430Q_For

<400>  100
gtctgacctc ccgtcagaac tcccgtacc                                          29

<210>  101
<211>  29
<212>  DNA
<213>  Artificial Sequence

<220>
<223>  Oligonucleotide SmuA_D430Q_Rev

<400>  101
ggtacgggag ttctgacggg aggtcagac                                          29

<210>  102
<211>  29
<212>  DNA
<213>  Artificial Sequence

<220>
<223>  Oligonucleotide SmuA_D430S_For

<400>  102
gtctgacctc ccgtagcaac tcccgtacc                                          29

<210>  103
<211>  29
<212>  DNA
<213>  Artificial Sequence

<220>
<223>  Oligonucleotide SmuA_D430S_Rev

<400>  103
ggtacgggag ttgctacggg aggtcagac                                          29

<210>  104
<211>  29
<212>  DNA
<213>  Artificial Sequence

<220>
<223>  Oligonucleotide SmuA_D430T_For

<400>  104
gtctgacctc ccgtaccaac tcccgtacc                                          29


<210>  105
<211>  29
<212>  DNA
<213>  Artificial Sequence

<220>
<223>  Oligonucleotide SmuA_D430T_Rev

<400>  105
ggtacgggag ttggtacggg aggtcagac                                          29


<210>  106
<211>  27
<212>  DNA
<213>  Artificial Sequence

<220>
<223>  Oligonucleotide SmuA_N431Q_For

<400>  106
acctcccgtg accaatcccg taccccg                                            27


<210>  107
<211>  27
<212>  DNA
<213>  Artificial Sequence

<220>
<223>  Oligonucleotide SmuA_N431Q_Rev

<400>  107
cggggtacgg gattggtcac gggaggt                                            27


<210>  108
<211>  27
<212>  DNA
<213>  Artificial Sequence

<220>
<223>  Oligonucleotide SmuA_N431S_For

<400>  108
acctcccgtg acagctcccg taccccg                                            27


<210>  109
<211>  27
<212>  DNA

<213>  Artificial Sequence

<220>
<223>  Oligonucleotide SmuA_N431S_Rev

<400>  109
cggggtacgg gagctgtcac gggaggt                                          27


<210>  110
<211>  27
<212>  DNA
<213>  Artificial Sequence

<220>
<223>  Oligonucleotide SmuA_F453L_For

<400>  110
ggtaaaccgt ggttgaaagt taacccg                                          27


<210>  111
<211>  27
<212>  DNA
<213>  Artificial Sequence

<220>
<223>  Oligonucleotide SmuA_F453L_Rev

<400>  111
cgggttaact ttcaaccacg gtttacc                                          27


<210>  112
<211>  27
<212>  DNA
<213>  Artificial Sequence

<220>
<223>  Oligonucleotide SmuA_F453W_For

<400>  112
ggtaaaccgt ggtggaaagt taacccg                                          27


<210>  113
<211>  27
<212>  DNA
<213>  Artificial Sequence

<220>
<223>  Oligonucleotide SmuA_F453W_Rev

<400>  113
cgggttaact ttccaccacg gtttacc                                          27


<210>  114
<211>  27
<212>  DNA
<213>  Artificial Sequence

<220>

<223>  Oligonucleotide SmuA_F453Y_For

<400>  114
ggtaaaccgt ggtacaaagt taacccg                                          27

<210>  115
<211>  27
<212>  DNA
<213>  Artificial Sequence

<220>
<223>  Oligonucleotide SmuA_F453Y_Rev

<400>  115
cgggttaact ttgtaccacg gtttacc                                          27

## Description

### FIELD OF THE INVENTION

The present invention relates to an isolated sucrose isomerase enzyme that comprises at least one point mutation that may be used to produce isomaltulose efficiently from a substrate comprising sucrose. In particular, the enzyme may comprise a combination of point mutations.

### BACKGROUND OF THE INVENTION

Isomaltulose is a sugar that is being used as a sucrose substitute in food and related products. Isomaltulose is known to be non-carcinogenic and has the same energy content as sucrose although it has a low glycemic index. Isomaltulose is also used in the production of Isomalt. Isomalt (also called, isomaltitol, Palatinit®) is a sugar substitute, which is obtained from sucrose. It is commonly used for production of sugar-free food products and especially useful for diabetic patients. It is produced in a two-stage process: first, sucrose is converted to isomaltulose (α-D-glucopyranosyl-1,6-fructose, also called Palatinose®) by rearrangement. The purified isomaltulose is then converted by catalytic hydrogenation to isomalt.

The isomerization of sucrose (cane sugar, beet sugar) to isomaltulose is as a rule carried out enzymatically with isomaltulose synthases (EC 5.4.99.11) (synonyms: saccharose mutase, sucrose isomerase, saccharose isomerase, sucrose mutase, and sucrose glucosylmutases). DE1049800, DE2217628, EP 28900, EP49472 and EP 91063 describe methods with immobilized bacterial cells for enzymatic conversion of sucrose to isomaltulose. For this, EP 0625578 uses bacterial strains from the group comprising *Protaminobacter rubrum* (CBS 574.77), *Serratia plymuthica* (ATCC 15928), *Serratia marcescens* (NCIB 8285), *Leuconostoc mesenteroides* (NRRL-B 512 F (ATCC 1083 a)) and *Erwinia rhapontici* (NCPPB 1578). EP 0392556 and EP1257638 describe the use of bacterial strains from the group comprising *Klebsiella terrigena* JCM 1687, *Klebsiella sp.* No. 88 (FERM BP-2838) and *Klebsiella singaporensis* LX3 and LX21. Their endogenic sucrose mutase(s) are known by the abbreviations SmuA, Pal I, SmtA, MutB, SIM and others.

These isomerization processes are carried out with live or dead cells, with immobilized or free cells: thus, DE3133123 and EP0915986 describe for example methods of immobilization of the enzyme catalysts with calcium alginate or ion exchangers, and EP0001099 describes a method with free, live cells, which can produce isomaltulose in the course of fermentation.

Something that all the known methods of isomerization have in common is that the sucrose is never completely converted to isomaltulose - not only traces of sucrose always be detected, other unwanted by-products such as trehalulose are also formed during the process of isomerisation of sucrose.

US8691535B2 discloses an improvement of enzyme SmuA from *Protaminobacter rubrum* that is used for the conversion of sucrose to isomaltulose. Use of the natural enzyme is limited to the production of around 85% of isomaltulose while the improved enzyme from US8691535B2 has product specificity of up to about 88% under optimized cultivation conditions. However, there is still room for improvement of the yield obtainable by the sucrose mutase SmuA so that more isomaltulose is formed. There is a need in the art to produce an improved sucrose mutase SmuA that is capable of producing more isomaltulose from the substrate sucrose and less by-products such as trehalulose, glucose and fructose.

### DESCRIPTION OF THE INVENTION

The present invention attempts to solve the problems above by providing a biotechnological means of producing a better yield of isomaltulose from sucrose with less by-products by genetically modifying the sucrose mutase SmuA. In particular, the sucrose mutase SmuA is genetically modified to comprise at least one point mutation or a combination of point mutations that enable the sucrose mutase SmuA to convert sucrose to produce more isomaltulose and less by-products compared to the sucrose mutases currently available for carrying out this process. The mutant enzyme may be transcribed in at least one cell to produce a microbial cell expressing the mutant sucrose mutase SmuA enzyme. This cell expressing the mutant sucrose mutase SmuA enzyme may be capable of producing isomaltulose from sucrose efficiently.

According to one aspect of the present invention, there is provided an isolated sucrose isomerase enzyme (EC 5.4.99.11) comprising at least one point mutation in SEQ ID NO: 1, wherein the point mutation is selected from the group consisting of Q181P, V216A, Y219L, I269V, F270Y, S277Q, S277T, D311P, T369F, D398G, L426V, L426Y, F453Y and combinations thereof.

The genetically modified sucrose isomerase enzyme according to any aspect of the present invention has improved specificity and selectivity for isomaltulose. Further, the sucrose isomerase enzyme according to any aspect of the present invention enables the sucrose isomerase enzyme and/or the cell expressing the sucrose isomerase enzyme to be used directly in the process of converting sucrose to isomaltulose without having to make costly modifications of reaction conditions and procedures that improve the efficiency of the process.

The genetically modified polypeptide sequence of the sucrose isomerase enzyme according to any aspect of the present invention may be directly obtained from the modification of a wild type sucrose isomerase sequence. In particular, the wild type sucrose isomerase sequence may be from *Serratia plymuthica.* Even more in particular, the base sucrose isomerase enzyme to which the point mutation(s) according to any aspect of the present invention are made may be SEQ ID NO:1. SEQ ID NO:1 may then be genetically modified to comprise at least one point mutation selected from the group consisting of Q181P, V216A, Y219L, I269V, F270Y, S277Q, S277T, D311P, T369F, D398G, L426V, L426Y, F453Y and combinations thereof. The presence of at least one of these point mutations in SEQ ID NO:1 enables the enzyme and the cell comprising the enzyme to be more efficient in producing isomaltulose from sucrose.

The sucrose isomerase enzyme (EC 5.4.99.11) is an enzyme that catalyzes the chemical reaction:

sucrose ⇆ isomaltulose

Isomaltulose is also known as 6-O-Alpha-D-Glucopyranosyl-D-Fructofuranose. The sucrose isomerase enzyme (EC 5.4.99.11) may also be called saccharose mutase, saccharose isomerase, sucrose mutase, isomaltulose synthetase, sucrose alpha-glucosyltransferase, trehalulose synthase, and sucrose glucosylmutases. Suitable isomaltulose synthases are for example those from *Enterobacter sp.* strain FMB1, *Erwinia rhapontici, Klebsiella planticola* strain UQ14S, *Klebsiella pneumoniae* NK33-98-8, *Klebsiella sp.* LX3, *Pantoea dispersa* UQ68J, *Protaminobacter ruber* Z12, *Protaminobacter rubrum, Pseudomonas mesoacidophila* MX-45, *Serratia plymuthica.* In particular, the sucrose isomerase may be from *Serratia plymuthica.* Even more in particular, the amino acid base sequence of sucrose isomerase from *Serratia plymuthica* that comprises at least one point mutation according to any aspect of the present invention comprises SEQ ID NO:5 (accession number D0VX20 from UniProt data bank).

In particular, the sucrose isomerase from *Serratia plymuthica* used according to any aspect of the present invention may comprise a polypeptide with sequence identity of at least 50, 60, 65, 70, 75, 80, 85, 90, 91, 94, 95, 98 or 100% to a polypeptide with SEQ ID NO:1. More in particular, the sucrose isomerase from *Serratia plymuthica* has a polypeptide sequence that comprises the amino acids of SEQ ID NO:1 that are essential for the function, for example the catalytic activity of a protein, or the fold or structure of the protein. The other amino acids may be deleted, substituted or replaced by insertions, or essential amino acids are replaced in a conservative manner to the effect that the biological activity of the sucrose isomerase from *Serratia plymuthica* is preserved. In particular, the sucrose isomerase from *Serratia plymuthica* according to any aspect of the present invention may comprise 70% sequence identity to SEQ ID NO: 1. More in particular, the sucrose isomerase from *Serratia plymuthica* comprises SEQ ID NO:1 with at the point mutation V465E that gives the enzyme a higher solubility relative to the wild type enzyme. In one example, the sucrose isomerase that may be used as a base sequence to which the point mutation(s) according to any aspect of the present invention is carried out may be SEQ ID NO:3 which is SEQ ID NO:1 with the point mutation V465E.

The term "enzyme" means any substance composed wholly or largely of protein or polypeptides that catalyzes or promotes, more or less specifically, one or more chemical or biochemical reactions. An example of an enzyme used according to any aspect of the present invention may be sucrose isomerase which catalyses the conversion of the substrate sucrose to isomaltulose. In particular, the sucrose isomerase SmuA, often known as sucrose mutase, is classified under the enzyme commission number (EC Number) EC 5.4.99.11. More in particular, the enzyme comprises at least the sequence of SEQ ID NO:1. In another example, the enzyme comprises at least the sequence of SEQ ID NO:3.

A "polypeptide" is a chain of chemical building blocks called amino acids that are linked together by chemical bonds called peptide bonds. A 'peptide' is made up of 10 or more amino acids. A protein or polypeptide, including an enzyme, may be "native" or "wild-type", meaning that it occurs in nature or has the amino acid sequence of a native protein, respectively. These terms are sometimes used interchangeably. A polypeptide may or may not be glycosylated. The term "wild type" as used herein in conjunction with a cell or microorganism may denote a cell with a genome make-up that is in a form as seen in nature. The term may be applicable for both the whole cell and for individual genes. The term 'wild type' may thus also include cells which have been genetically modified in other aspects (i.e. with regard to one or more genes) but not in relation to the genes of interest. The term "wild type" therefore does not include such cells where the gene sequences of the specific genes of interest have been altered at least partially by man using recombinant methods. A wild type cell according to any aspect of the present invention thus refers to a cell that has no genetic mutation with respect to the whole genome and/or a particular gene. Therefore, in one example, a wild type of sucrose isomerase SmuA may be an enzyme that is found in nature without any of the specific mutations according to any aspect of the present invention. In another example, the wild type cell relative to the mutant sucrose isomerase SmuA according to any aspect of the present invention relates to a cell that has the natural, non-altered expression of sucrose isomerase SmuA with mutations that may occur naturally but not at amino acid positions 181, 216, 219, 269, 270, 277, 277, 311, 369, 398, 426, 426, 453 and 465. In fact the wild type sequence of sucrose isomerase SmuA from *Serratia plymuthica* used according to any aspect of the present invention refers to SEQ ID NO:1. The wild type sequence of sucrose isomerase SmuA from *Serratia plymuthica* used according to any aspect of the present invention may also comprise a signal sequence, SEQ ID NO:2.

A "variant" of SmuA or at least SEQ ID NO:1, as used herein, refers to an amino acid sequence that is altered by one or more amino acids. The variant may have "conservative" changes, wherein a substituted amino acid has similar structural or chemical properties (e.g., replacement of leucine with isoleucine). More rarely, a variant may have "non-conservative" changes (e.g., replacement of glycine with tryptophan). Analogous minor variations may also include amino acid deletions or insertions, or both. Guidance in determining which amino acid residues may be substituted, inserted, or deleted without abolishing biological or immunological activity may be found using computer programs well known in the art, for example, DNASTAR , Clustal Omega or PyMOL software. According to any aspect of the present invention, the variant of SEQ ID NO:1 used as the wild type SmuA may not comprise a mutation at amino acid positions 181, 216, 219, 269, 270, 277, 277, 311, 369, 398, 426, 426, 453 and/or 465.

A polypeptide may also be a "mutant" meaning that it has been made, altered, derived, or is in some way different or changed from its wild-type form, or from another mutant. Mutant proteins typically have amino acid substitutions at one or more positions. Mutant DNA molecules typically have nucleotide substitutions in one or more positions. Mutant forms of a protein or DNA molecule can have the same, or altered, functions in comparison to the wild-type. For simplification, mutants may be referred to by their variation from the single amino acid code from which the mutation arose. For example, in one format the mutant is referred to as XPOSY, where "X" refers to the single letter code of the amino acid in the original sequence, "POS" refers to the position of the mutation in the sequence, and Y refers to the single letter code for the new amino acid appearing at the mutation's position. For example, V465E would mean that in the original protein, the amino acid at position 465 is a valine ("V"), but in the mutant, the valine is replaced with a glutamic acid ("E").

According to any aspect of the present invention, the SmuA base or foundation sequence that is used to introduce the point mutation(s) according to any aspect of the present invention may comprise a polypeptide with sequence identity of at least 50, 60, 65, 70, 75, 80, 85, 90, 91, 94, 95, 98 or 100% to a polypeptide SEQ ID NO:3. More in particular, the SmuA base or foundation sequence that is used to introduce the point mutation(s) according to any aspect of the present invention comprises SEQ ID NO:3.

In particular, the mutant SmuA enzyme according to any aspect of the present invention may comprise at least one point mutation at amino acid positions 181, 216, 219, 269, 270, 277, 277, 311, 369, 398, 426, 426, 453 and 465 of the SmuA enzyme, particularly comprising SEQ ID NO:1 or 3. More in particular, the point mutations may be at amino acid positions 219, 369, 398, and/or 453. Even more in particular, the point mutations may be selected from the group consisting of Q181P, V216A, Y219L, I269V, F270Y, S277Q, S277T, D311P, T369F, D398G, L426V, L426Y, F453Y and combinations thereof. In particular, the point mutations may be selected from the group consisting of Y219L, T369F, D398G, F453Y and combinations thereof.

Any techniques in molecular biology can be used for producing SmuA enzymes with specific point mutations. The term 'point mutation', or single base modification, is a type of mutation that causes a single nucleotide base change, insertion, or deletion of the genetic material, DNA or RNA. In one example, point mutation may refer to an amino acid substitution in a wild type amino acid sequence of SmuA. Some examples of methods that may be used include error-prone polymerase chain reaction, Kunkel mutagenesis (Kunkel, T. A. (1985). Proc Natl Acad Sci USA, 82(2), 488-92;), QuikChange (QuikChange Multi Site-Directed Mutagenesis Kit, Instruction Manual, Catalog #200514, Revision #124003a, Stratagene; QuikChange Site-Directed Mutagenesis Kit, Stratagene), inverse PCR (Dominy C N, (2003), Methods Mol Biol 235:209-23;), cassette mutagenesis (in which the specific region optimized is replaced with a synthetically mutagenized oligonucleotide), oligonucleotide-directed mutagenesis, parallel PCR (which uses a large number of different PCR reactions that occur in parallel in the same vessel, such that the product of one reaction primes the product of another reaction), random mutagenesis (e.g. by random fragmentation and reassembly of the fragments by mutual priming), site-specific mutations, parallel PCR (e.g. recombination on a pool of DNA sequences), sexual PCR, chemical mutagenesis (e.g. by using sodium bisulfite, nitrous acid, hydroxylamine, hydrazine, formic acid or by adding intercalating agents such as proflavine, acriflavine, quinacrine), irradiation (X-rays or ultraviolet light, and/or subjecting the polynucleotide to propagation in a host cell that is deficient in normal DNA damage repair function), or DNA shuffling (e.g. *in vitro* or *in vivo* homologous recombination of pools of nucleic acid fragments or polynucleotides). Any one of these techniques can also be employed under low-fidelity polymerization conditions to introduce a low level of point mutations randomly over a long sequence, or to mutagenize a mixture of fragments of unknown sequence.

Any of the enzymes used according to any aspect of the present invention, may be an isolated enzyme. In particular, the enzymes used according to any aspect of the present invention may be used in an active state and in the presence of all cofactors, substrates, auxiliary and/or activating polypeptides or factors essential for its activity. The term "isolated", as used herein, means that the enzyme of interest is enriched compared to the cell in which it occurs naturally. The enzyme may be enriched by chromatographic purification or precipitation procedures. For example, the enzyme of interest may constitute more than 5, 10, 20, 50, 75, 80, 85, 90, 95 or 99 percent of all the polypeptides present in the preparation as judged by visual inspection of an SDS polyacrylamide gel following staining with Coomassie blue dye.

In another example, the point mutation may be at amino acid positions 398 and 453, 269 and 453, 270 and 453, 219 and 398, 181 and 269, 181 and 398, 369 and 398 or 219 and 453. In particular, the enzyme according to any aspect of the present invention may comprise a double mutation on SEQ ID NO:1 that comprises at least two point mutations selected from the group consisting of Q181P and V216A, Q181P and Y219L, Q181P and I269V, Q181P and F270Y, Q181P and S277Q, Q181P and S277T, Q181P and D311P, Q181P and T369F, Q181P and D398G, Q181P and L426V, Q181P and L426Y, Q181P and F453Y, V216A and Y219L, V216A and I269V, V216A and F270Y, V216A and S277Q, V216A and S277T, V216A and D311P, V216A and T369F, V216A and D398G, V216A and L426V, V216A and L426Y, V216A and F453Y, Y219L and I269V, Y219L and F270Y, Y219L and S277Q, Y219L and S277T, Y219L and D311P, Y219L and T369F, Y219L and D398G, Y219L and L426V, Y219L and L426Y, Y219L and F453Y, I269V and F270Y, I269V and S277Q, I269V and S277T, I269V and D311P, I269V and T369F, I269V and D398G, I269V and L426V, I269V and L426Y, I269V and F453Y, F270Y and S277Q, F270Y and S277T, F270Y and D311P, F270Y and T369F, F270Y and D398G, F270Y and L426V, F270Y and L426Y, F270Y and F453Y, S277Q and D311P, S277Q and D311P, S277Q and T369F, S277Q and D398G, S277Q and L426V, S277Q and L426Y, S277Q and F453Y, S277T and D311P, S277T and T369F, S277T and D398G, S277T and L426V, S277T and L426Y, S277T and F453Y, D311P and T369F, D311P and D398G, D311P and L426V, D311P and L426Y, D311P and F453Y, T369F and D398G, T369F and L426V, T369F and L426Y, T369F and F453Y, D398G and L426V, D398G and L426Y, D398G and F453Y, L426V and F453Y, and L426Y and F453Y. More in particular, the double mutation may be comprise at least two point mutations on SEQ ID NO:1 selected from the group consisting of D398G and F453Y, I269V and F453Y, F270Y and F453Y, Y219L and D398G, Q181P and I269V, Q181P and D398G, T369F and D398G and Y219L and F453Y.

In another example, the point mutation may be at amino acid positions 398 and 453, 269 and 453, 270 and 453, 219 and 398, 181 and 269, 181 and 398, 369 and 398 or 219 and 453. In particular, the enzyme according to any aspect of the present invention may comprise a double mutation on SEQ ID NO:3 that comprises at least two point mutations selected from the group consisting of Q181P and V216A, Q181P and Y219L, Q181P and I269V, Q181P and F270Y, Q181P and S277Q, Q181P and S277T, Q181P and D311P, Q181P and T369F, Q181P and D398G, Q181P and L426V, Q181P and L426Y, Q181P and F453Y, V216A and Y219L, V216A and I269V, V216A and F270Y, V216A and S277Q, V216A and S277T, V216A and D311P, V216A and T369F, V216A and D398G, V216A and L426V, V216A and L426Y, V216A and F453Y, Y219L and I269V, Y219L and F270Y, Y219L and S277Q, Y219L and S277T, Y219L and D311P, Y219L and T369F, Y219L and D398G, Y219L and L426V, Y219L and L426Y, Y219L and F453Y, I269V and F270Y, I269V and S277Q, I269V and S277T, I269V and D311P, I269V and T369F, I269V and D398G, I269V and L426V, I269V and L426Y, I269V and F453Y, F270Y and S277Q, F270Y and S277T, F270Y and D311P, F270Y and T369F, F270Y and D398G, F270Y and L426V, F270Y and L426Y, F270Y and F453Y, S277Q and D311P, S277Q and D311P, S277Q and T369F, S277Q and D398G, S277Q and L426V, S277Q and L426Y, S277Q and F453Y, S277T and D311P, S277T and T369F, S277T and D398G, S277T and L426V, S277T and L426Y, S277T and F453Y, D311P and T369F, D311P and D398G, D311P and L426V, D311P and L426Y, D311P and F453Y, T369F and D398G, T369F and L426V, T369F and L426Y, T369F and F453Y, D398G and L426V, D398G and L426Y, D398G and F453Y, L426V and F453Y, and L426Y and F453Y. More in particular, the double mutation may be comprise at least two point mutations on SEQ ID NO:3 selected from the group consisting of D398G and F453Y, I269V and F453Y, F270Y and F453Y, Y219L and D398G, Q181P and I269V, Q181P and D398G, T369F and D398G and Y219L and F453Y.

In a further example, the enzyme according to any aspect of the present invention may comprise a triple mutation on SEQ ID NO:1 that comprises at least three point mutations selected from the group consisting of Q181P, V216A, and Y219L, Q181P, V216A, and I269V, Q181P, V216A, and F270Y, Q181P, V216A, and S277Q, Q181P, V216A, and S277T, Q181P, V216A, and D311P, Q181P, V216A, and T369F, Q181P, V216A, and D398G, Q181P, V216A, and L426V, Q181P, V216A, and L426Y, Q181P, V216A, and F453Y, Q181P, Y219L, and I269V, Q181P, Y219L, and F270Y, Q181P, Y219L, and S277Q, Q181P, Y219L, and S277T, Q181P, Y219L, and D311P, Q181P, Y219L, and T369F, Q181P, Y219L, and D398G, Q181P, Y219L, and L426V, Q181P, Y219L, and L426Y, Q181P, Y219L, and F453Y, Q181P, I269V and F270Y, Q181P, I269V and S277Q, Q181P, I269V and S277T, Q181P, I269V and D311P, Q181P, I269V and T369F, Q181P, I269V and D398G, Q181P, I269V and L426V, Q181P, I269V and L426Y, Q181P, I269V and F453Y, Q181P, F270Y and S277Q, Q181P, F270Y and S277T, Q181P, F270Y and D311P, Q181P, F270Y and T369F, Q181P, F270Y and D398G, Q181P, F270Y and L426V, Q181P, F270Y and L426Y, Q181P, F270Y and F453Y, Q181P, S277Q and D311P, Q181P, S277Q and T369F, Q181P, S277Q and D398G, Q181P, S277Q and L426V, Q181P, S277Q and L426Y, Q181P, S277Q and F453Y, Q181P, S277T and D311P, Q181P, S277T and T369F, Q181P, S277T and D398G, Q181P, S277T and L426V, Q181P, S277T and L426Y, Q181P, S277T and F453Y, Q181P, D311P and T369F, Q181P, D311P and D398G, Q181P, D311P and L426V, Q181P, D311P and L426Y, Q181P, D311P and F453Y, Q181P, T369F and D398G, Q181P, T369F and L426V, Q181P, T369F and L426Y, Q181P, T369F and F453Y, Q181P, D398G, and L426V, Q181P, D398G, and L426Y, Q181P, D398G, and F453Y, Q181P, L426V and F453Y, V216A, Y219L and I269V, V216A, Y219L and F270Y, V216A, Y219L and S277Q, V216A, Y219L and S277T, V216A, Y219L and D311P, V216A, Y219L and T369F, V216A, Y219L and D398G, V216A, Y219L and L426V, V216A, Y219L and L426Y, V216A, Y219L and F453Y, V216A, I269V and F270Y, V216A, I269V and S277Q, V216A, I269V and S277T, V216A, I269V and D311P, V216A, I269V and T369F, V216A, I269V and D398G, V216A, I269V and L426V, V216A, I269V and L426Y, V216A, I269V and F453Y, V216A, F270Y and S277Q, V216A, F270Y and S277T, V216A, F270Y and D311P, V216A, F270Y and T369F, V216A, F270Y and D398G, V216A, F270Y and L426V, V216A, F270Y and L426Y, V216A, F270Y and F453Y, V216A, S277Q and D311P, V216A, S277Q and T369F, V216A, S277Q and D398G, V216A, S277Q and L426V, V216A, S277Q and L426Y, V216A, S277Q and F453Y, V216A, S277T and D311P, V216A, S277T and T369F, V216A, S277T and D398G, V216A, S277T and L426V, V216A, S277T and L426Y, V216A, S277T and F453Y, V216A, D311P and T369F, V216A, D311P and D398G, V216A, D311P and L426V, V216A, D311P and L426Y, V216A, D311P and F453Y, V216A, T369F and D398G, V216A, T369F and L426V, V216A, T369F and L426Y, V216A, T369F and F453Y, V216A, D398G and L426V, V216A, D398G and L426Y, V216A, D398G and F453Y, V216A, L426V and F453Y, V216A, L426Y and F453Y, Y219L, I269V and F270Y, Y219L, I269V and S277Q, Y219L, I269V and S277T, Y219L, I269V and D311P, Y219L, I269V and T369F, Y219L, I269V and D398G, Y219L, I269V and L426V, Y219L, I269V and L426Y, Y219L, I269V and F453Y, Y219L, F270Y and S277Q, Y219L, F270Y and S277T, Y219L, F270Y and D311P, Y219L, F270Y and T369F, Y219L, F270Y and D398G, Y219L, F270Y and L426V, Y219L, F270Y and L426Y, Y219L, F270Y and F453Y, Y219L, S277Q and D311P, Y219L, S277Q and T369F, Y219L, S277Q and D398G, Y219L, S277Q and L426V, Y219L, S277Q and L426Y, Y219L, S277Q and F453Y, Y219L, S277T, and D311P, Y219L, S277T, and T369F, Y219L, S277T, and D398G, Y219L, S277T, and L426V, Y219L, S277T, and L426Y, Y219L, S277T, and F453Y, Y219L, D311P and T369F, Y219L, D311P and D398G, Y219L, D311P and L426V, Y219L, D311P and L426Y, Y219L, D311P and F453Y, Y219L, T369F and D398G, Y219L, T369F and L426V, Y219L, T369F and L426Y, Y219L, T369F and F453Y, Y219L, D398G and L426V, Y219L, D398G and L426Y, Y219L, D398G and F453Y, Y219L, L426V and F453Y, Y219L, L426Y and F453Y, I269V, F270Y and S277Q, I269V, F270Y and S277T, I269V, F270Y and D311P, I269V, F270Y and T369F, I269V, F270Y and D398G, I269V, F270Y and L426V, I269V, F270Y and L426Y, I269V, F270Y and F453Y, I269V, S277Q and D311P, I269V, S277Q and T369F, I269V, S277Q and D398G, I269V, S277Q and L426V, I269V, S277Q and L426Y, I269V, S277Q and F453Y, I269V, S277T and D311P, I269V, S277T and T369F, I269V, S277T and D398G, I269V, S277T and L426V, I269V, S277T and L426Y, I269V, S277T and F453Y, I269V, D311P and T369F, I269V, D311P and D398G, I269V, D311P and L426V, I269V, D311P and L426Y, I269V, D311P and F453Y, I269V, T369F and D398G, I269V, T369F and L426V, I269V, T369F and L426Y, I269V, T369F and F453Y, I269V, D398G and L426V, I269V, D398G and L426Y, I269V, D398G and F453Y, I269V, L426V and L426Y, I269V, L426V and F453Y, I269V, L426Y and F453Y, F270Y, S277Q and D311P, F270Y, S277Q and T369F, F270Y, S277Q and D398G, F270Y, S277Q and L426V, F270Y, S277Q and L426Y, F270Y, S277Q and F453Y, F270Y, S277T and D311P, F270Y, S277T and T369F, F270Y, S277T and D398G, F270Y, S277T and L426V, F270Y, S277T and L426Y, F270Y, S277T and L426Y, F270Y, D311P and T369F, F270Y, D311P and D398G, F270Y, D311P and L426V, F270Y, D311P and L426Y, F270Y, D311P and F453Y, F270Y, T369F and D398G, F270Y, T369F and L426V, F270Y, T369F and L426Y, F270Y, T369F and F453Y, F270Y, D398G and L426V, F270Y, D398G and L426Y, F270Y, D398G and F453Y, F270Y, L426V and F453Y, F270Y, L426Y and F453Y, S277Q, D311P and T369F, S277Q, D311P and D398G, S277Q, D311P and L426V, S277Q, D311P and L426Y, S277Q, D311P and F453Y, S277Q, T369F and D398G, S277Q, T369F and L426V, S277Q, T369F and L426Y, S277Q, T369F and F453Y, S277Q, D398G and L426V, S277Q, D398G and L426Y, S277Q, D398G and F453Y, S277Q, L426V and F453Y, S277Q, L426Y and F453Y, S277T, D311P and T369F, S277T, D311P and D398G, S277T, D311P and L426V, S277T, D311P and L426Y, S277T, D311P and F453Y, S277T, T369F and D398G, S277T, T369F and L426V, S277T, T369F and L426Y, S277T, T369F and F453Y, S277T, D398G and L426V, S277T, D398G and L426Y, S277T, D398G and F453Y, S277T, L426V and F453Y, S277T, L426Y and F453Y, D311P, T369F and D398G, D311P, T369F and L426V, D311P, T369F and L426Y, D311P, T369F and F453Y, D311P, D398G and L426V, D311P, D398G and L426Y, D311P, D398G and F453Y, D311P, L426V and F453Y, D311P, L426Y and F453Y, T369F, D398G and L426V, T369F, D398G and L426Y, T369F, D398G and F453Y, T369F, L426V and F453Y, T369F, L426Y and F453Y, D398G, L426V and F453Y, D398G, L426Y and F453Y, and combinations thereof. More in particular, the triple mutation may be comprise at least three point mutations on SEQ ID NO:1 selected from the group consisting of Q181P, D398G and F453Y, T369F, D398G and F453Y and T396F, D398G and L426Y.

In one example, the enzyme according to any aspect of the present invention may comprise a triple mutation on SEQ ID NO:3 that comprises at least three point mutations selected from the group consisting of Q181P, V216A, and Y219L, Q181P, V216A, and I269V, Q181P, V216A, and F270Y, Q181P, V216A, and S277Q, Q181P, V216A, and S277T, Q181P, V216A, and D311P, Q181P, V216A, and T369F, Q181P, V216A, and D398G, Q181P, V216A, and L426V, Q181P, V216A, and L426Y, Q181P, V216A, and F453Y, Q181P, Y219L, and I269V, Q181P, Y219L, and F270Y, Q181P, Y219L, and S277Q, Q181P, Y219L, and S277T, Q181P, Y219L, and D311P, Q181P, Y219L, and T369F, Q181P, Y219L, and D398G, Q181P, Y219L, and L426V, Q181P, Y219L, and L426Y, Q181P, Y219L, and F453Y, Q181P, I269V and F270Y, Q181P, I269V and S277Q, Q181P, I269V and S277T, Q181P, I269V and D311P, Q181P, I269V and T369F, Q181P, I269V and D398G, Q181P, I269V and L426V, Q181P, I269V and L426Y, Q181P, I269V and F453Y, Q181P, F270Y and S277Q, Q181P, F270Y and S277T, Q181P, F270Y and D311P, Q181P, F270Y and T369F, Q181P, F270Y and D398G, Q181P, F270Y and L426V, Q181P, F270Y and L426Y, Q181P, F270Y and F453Y, Q181P, S277Q and D311P, Q181P, S277Q and T369F, Q181P, S277Q and D398G, Q181P, S277Q and L426V, Q181P, S277Q and L426Y, Q181P, S277Q and F453Y, Q181P, S277T and D311P, Q181P, S277T and T369F, Q181P, S277T and D398G, Q181P, S277T and L426V, Q181P, S277T and L426Y, Q181P, S277T and F453Y, Q181P, D311P and T369F, Q181P, D311P and D398G, Q181P, D311P and L426V, Q181P, D311P and L426Y, Q181P, D311P and F453Y, Q181P, T369F and D398G, Q181P, T369F and L426V, Q181P, T369F and L426Y, Q181P, T369F and F453Y, Q181P, D398G, and L426V, Q181P, D398G, and L426Y, Q181P, D398G, and F453Y, Q181P, L426V and F453Y, V216A, Y219L and I269V, V216A, Y219L and F270Y, V216A, Y219L and S277Q, V216A, Y219L and S277T, V216A, Y219L and D311P, V216A, Y219L and T369F, V216A, Y219L and D398G, V216A, Y219L and L426V, V216A, Y219L and L426Y, V216A, Y219L and F453Y, V216A, I269V and F270Y, V216A, I269V and S277Q, V216A, I269V and S277T, V216A, I269V and D311P, V216A, I269V and T369F, V216A, I269V and D398G, V216A, I269V and L426V, V216A, I269V and L426Y, V216A, I269V and F453Y, V216A, F270Y and S277Q, V216A, F270Y and S277T, V216A, F270Y and D311P, V216A, F270Y and T369F, V216A, F270Y and D398G, V216A, F270Y and L426V, V216A, F270Y and L426Y, V216A, F270Y and F453Y, V216A, S277Q and D311P, V216A, S277Q and T369F, V216A, S277Q and D398G, V216A, S277Q and L426V, V216A, S277Q and L426Y, V216A, S277Q and F453Y, V216A, S277T and D311P, V216A, S277T and T369F, V216A, S277T and D398G, V216A, S277T and L426V, V216A, S277T and L426Y, V216A, S277T and F453Y, V216A, D311P and T369F, V216A, D311P and D398G, V216A, D311P and L426V, V216A, D311P and L426Y, V216A, D311P and F453Y, V216A, T369F and D398G, V216A, T369F and L426V, V216A, T369F and L426Y, V216A, T369F and F453Y, V216A, D398G and L426V, V216A, D398G and L426Y, V216A, D398G and F453Y, V216A, L426V and F453Y, V216A, L426Y and F453Y, Y219L, I269V and F270Y, Y219L, I269V and S277Q, Y219L, I269V and S277T, Y219L, I269V and D311P, Y219L, I269V and T369F, Y219L, I269V and D398G, Y219L, I269V and L426V, Y219L, I269V and L426Y, Y219L, I269V and F453Y, Y219L, F270Y and S277Q, Y219L, F270Y and S277T, Y219L, F270Y and D311P, Y219L, F270Y and T369F, Y219L, F270Y and D398G, Y219L, F270Y and L426V, Y219L, F270Y and L426Y, Y219L, F270Y and F453Y, Y219L, S277Q and D311P, Y219L, S277Q and T369F, Y219L, S277Q and D398G, Y219L, S277Q and L426V, Y219L, S277Q and L426Y, Y219L, S277Q and F453Y, Y219L, S277T, and D311P, Y219L, S277T, and T369F, Y219L, S277T, and D398G, Y219L, S277T, and L426V, Y219L, S277T, and L426Y, Y219L, S277T, and F453Y, Y219L, D311P and T369F, Y219L, D311P and D398G, Y219L, D311P and L426V, Y219L, D311P and L426Y, Y219L, D311P and F453Y, Y219L, T369F and D398G, Y219L, T369F and L426V, Y219L, T369F and L426Y, Y219L, T369F and F453Y, Y219L, D398G and L426V, Y219L, D398G and L426Y, Y219L, D398G and F453Y, Y219L, L426V and F453Y, Y219L, L426Y and F453Y, I269V, F270Y and S277Q, I269V, F270Y and S277T, I269V, F270Y and D311P, I269V, F270Y and T369F, I269V, F270Y and D398G, I269V, F270Y and L426V, I269V, F270Y and L426Y, I269V, F270Y and F453Y, I269V, S277Q and D311P, I269V, S277Q and T369F, I269V, S277Q and D398G, I269V, S277Q and L426V, I269V, S277Q and L426Y, I269V, S277Q and F453Y, I269V, S277T and D311P, I269V, S277T and T369F, I269V, S277T and D398G, I269V, S277T and L426V, I269V, S277T and L426Y, I269V, S277T and F453Y, I269V, D311P and T369F, I269V, D311P and D398G, I269V, D311P and L426V, I269V, D311P and L426Y, I269V, D311P and F453Y, I269V, T369F and D398G, I269V, T369F and L426V, I269V, T369F and L426Y, I269V, T369F and F453Y, I269V, D398G and L426V, I269V, D398G and L426Y, I269V, D398G and F453Y, I269V, L426V and L426Y, I269V, L426V and F453Y, I269V, L426Y and F453Y, F270Y, S277Q and D311P, F270Y, S277Q and T369F, F270Y, S277Q and D398G, F270Y, S277Q and L426V, F270Y, S277Q and L426Y, F270Y, S277Q and F453Y, F270Y, S277T and D311P, F270Y, S277T and T369F, F270Y, S277T and D398G, F270Y, S277T and L426V, F270Y, S277T and L426Y, F270Y, S277T and L426Y, F270Y, D311P and T369F, F270Y, D311P and D398G, F270Y, D311P and L426V, F270Y, D311P and L426Y, F270Y, D311P and F453Y, F270Y, T369F and D398G, F270Y, T369F and L426V, F270Y, T369F and L426Y, F270Y, T369F and F453Y, F270Y, D398G and L426V, F270Y, D398G and L426Y, F270Y, D398G and F453Y, F270Y, L426V and F453Y, F270Y, L426Y and F453Y, S277Q, D311P and T369F, S277Q, D311P and D398G, S277Q, D311P and L426V, S277Q, D311P and L426Y, S277Q, D311P and F453Y, S277Q, T369F and D398G, S277Q, T369F and L426V, S277Q, T369F and L426Y, S277Q, T369F and F453Y, S277Q, D398G and L426V, S277Q, D398G and L426Y, S277Q, D398G and F453Y, S277Q, L426V and F453Y, S277Q, L426Y and F453Y, S277T, D311P and T369F, S277T, D311P and D398G, S277T, D311P and L426V, S277T, D311P and L426Y, S277T, D311P and F453Y, S277T, T369F and D398G, S277T, T369F and L426V, S277T, T369F and L426Y, S277T, T369F and F453Y, S277T, D398G and L426V, S277T, D398G and L426Y, S277T, D398G and F453Y, S277T, L426V and F453Y, S277T, L426Y and F453Y, D311P, T369F and D398G, D311P, T369F and L426V, D311P, T369F and L426Y, D311P, T369F and F453Y, D311P, D398G and L426V, D311P, D398G and L426Y, D311P, D398G and F453Y, D311P, L426V and F453Y, D311P, L426Y and F453Y, T369F, D398G and L426V, T369F, D398G and L426Y, T369F, D398G and F453Y, T369F, L426V and F453Y, T369F, L426Y and F453Y, D398G, L426V and F453Y, D398G, L426Y and F453Y, and combinations thereof. More in particular, the triple mutation may be comprise at least three point mutations on SEQ ID NO:3 selected from the group consisting of Q181P, D398G and F453Y, T369F, D398G and F453Y and T396F, D398G and L426Y.

In a further example, the enzyme according to any aspect of the present invention may comprise a quadruple mutation on SEQ ID NO:1 that comprises at least four point mutations selected from the group consisting of Q181P, V216A, Y219L, I269V, F270Y, S277Q, S277T, D311P, T369F, D398G, L426V, L426Y, and F453Y. In particular, the quadruple mutation comprises at least T369F and D398G. More in particular, the quadruple mutation may be selected from the group consisting of T396F, D398G, F453Y and Y219L, T369F, D398G, L426Y and I269V, T369F, D398G, L426Y and Y219L, T369F, D398G, L426Y and L426Y and T369F, D398G, L426Y and F453Y carried out on SEQ ID NO:1.

In one example, the enzyme according to any aspect of the present invention may comprise a quadruple mutation on SEQ ID NO:3 that comprises at least four point mutations selected from the group consisting of Q181P, V216A, Y219L, I269V, F270Y, S277Q, S277T, D311P, T369F, D398G, L426V, L426Y, and F453Y. In particular, the quadruple mutation comprises at least T369F and D398G. More in particular, the quadruple mutation may be selected from the group consisting of T396F, D398G, F453Y and Y219L, T369F, D398G, L426Y and I269V, T369F, D398G, L426Y and Y219L, T369F, D398G, L426Y and L426Y and T369F, D398G, L426Y and F453Y carried out on SEQ ID NO:3.

The enzyme according to any aspect of the present invention may be immobilized on a support. The support may be a filter membrane.

According to one aspect of the present invention, there is provided an isolated nucleic acid comprising a polynucleotide sequence that encodes the enzyme according to any aspect of the present invention.

According to another aspect of the present invention, there is provided an isolated cell comprising a vector comprising the nucleic acid according to any aspect of the present invention. Examples of suitable vectors may also be gene editing systems that are disclosed for example in US8697359B1. In particular, the vector may be a CRISPR/ CAS vector.

According to a further aspect of the present invention, there is provided a microbial cell expressing a mutant sucrose isomerase enzyme, the mutant sucrose isomerase enzyme comprising at least one point mutation in SEQ ID NO: 1, wherein the point mutation is selected from the group consisting of Q181P, V216A, Y219L, I269V, F270Y, S277Q, S277T, D311P, T369F, D398G, L426V, L426Y, F453Y and combinations thereof.

According to yet another aspect of the present invention, there is provided a microbial cell expressing a mutant sucrose isomerase enzyme, the mutant sucrose isomerase enzyme comprising at least one point mutation in SEQ ID NO: 3, wherein the point mutation is selected from the group consisting of Q181P, V216A, Y219L, I269V, F270Y, S277Q, S277T, D311P, T369F, D398G, L426V, L426Y, F453Y and combinations thereof.

According to any aspect of the present invention, the microbial cell may be selected from the species of bacteria from the group consisting of, *Abiotrophia, Acaryochloris, Accumulibacter, Acetivibrio, Acetobacter, Acetohalobium, Acetonema, Achromobacter, Acidaminococcus, Acidimicrobium, Acidiphilium, Acidithiobacillus, Acidobacterium, Acidothermus, Acidovorax, Acinetobacter, Actinobacillus, Actinomyces, Actinosynnema, Aerococcus, Aeromicrobium, Aeromonas, Afipia, Aggregatibacter, Agrobacterium, Ahrensia, Akkermansia, Alcanivorax, Alicycliphilus, Alicyclobacillus, Aliivibrio, Alkalilimnicola, Alkaliphilus, Allochromatium, Alteromonadales, Alteromonas, Aminobacterium, Aminomonas, Ammonifex, Amycolatopsis, Amycolicicoccus, Anabaena, Anaerobaculum, Anaerococcus, Anaerofustis, Anaerolinea, Anaeromyxobacter, Anaerostipes, Anaerotruncus, Anaplasma, Anoxybacillus, Aquifex, Arcanobacterium, Arcobacter, Aromatoleum, Arthrobacter, Arthrospira, Asticcacaulis, Atopobium, Aurantimonas, Azoarcus, Azorhizobium, Azospirillum, Azotobacter, Bacillus, Bartonella, Basfia, Baumannia, Bdellovibrio, Beggiatoa, Beijerinckia, Bermanella, Beutenbergia, Bifidobacterium, Bilophila, Blastopirellula, Blautia, Blochmannia, Bordetella, Borrelia, Brachybacterium, Brachyspira, Bradyrhizobium, Brevibacillus, Brevibacterium, Brevundimonas, Brucella, Buchnera, Bulleidia, Burkholderia, Butyrivibrio, Caldalkalibacillus, Caldanaerobacter, Caldicellulosiruptor, Calditerrivibrio, Caminibacter, Campylobacter, Carboxydibrachium, Carboxydothermus, Cardiobacterium, Carnobacterium, Carsonella, Catenibacterium, Catenulispora, Catonella, Caulobacter, Cellulomonas, Cellvibrio, Centipeda, Chelativorans, Chloroflexus, Chromobacterium, Chromohalobacter, Chthoniobacter, Citreicella, Citrobacter, Citromicrobium, Clavibacter, Cloacamonas, Clostridium, Collinsella, Colwellia, Comamonas, Conexibacter, Congregibacter, Coprobacillus, Coprococcus, Coprothermobacter, Coraliomargarita, Coriobacterium, corrodens, Corynebacterium, Coxiella, Crocosphaera, Cronobacter, Cryptobacterium, Cupriavidus, Cyanobium, Cyanothece, Cylindrospermopsis, Dechloromonas, Deferribacter, Dehalococcoides, Dehalogenimonas, Deinococcus, Delftia, Denitrovibrio, Dermacoccus, Desmospora, Desulfarculus, Desulphateibacillum, Desulfitobacterium, Desulfobacca, Desulfobacterium, Desulfobulbus, Desulfococcus, Desulfohalobium, Desulfomicrobium, Desulfonatronospira, Desulforudis, Desulfotalea, Desulfotomaculum, Desulfovibrio, Desulfurispirillum, Desulfurobacterium, Desulfuromonas, Dethiobacter, Dethiosulfovibrio, Dialister, Dichelobacter, Dickeya, Dictyoglomus, Dietzia, Dinoroseobacter, Dorea, Edwardsiella, Ehrlichia, Eikenella, Elusimicrobium, Endoriftia, Enhydrobacter, Enterobacter, Enterococcus, Epulopiscium, Erwinia, Erysipelothrix, Erythrobacter, Escherichia, Ethanoligenens, Eubacterium, Eubacterium, Exiguobacterium, Faecalibacterium, Ferrimonas, Fervidobacterium, Fibrobacter, Finegoldia, Flexistipes, Francisella, Frankia, Fructobacillus, Fulvimarina, Fusobacterium, Gallibacterium, Gallionella, Gardnerella, Gemella, Gemmata, Gemmatimonas, Geobacillus, Geobacter, Geodermatophilus, Glaciecola, Gloeobacter, Glossina, Gluconacetobacter, Gordonia, Granulibacter, Granulicatella, Grimontia, Haemophilus, Hahella, Halanaerobiumns, Haliangium, Halomonas, Halorhodospira, Halothermothrix, Halothiobacillus, Hamiltonella, Helicobacter, Heliobacterium, Herbaspirillum, Herminiimonas, Herpetosiphon, Hippea, Hirschia, Histophilus, Hodgkinia, Hoeflea, Holdemania, Hydrogenivirga, Hydrogenobaculum, Hylemonella, Hyphomicrobium, Hyphomonas, Idiomarina, Ilyobacter, Intrasporangium, Isoptericola, Isosphaera, Janibacter, Janthinobacterium, Jonesia, Jonquetella, Kangiella, Ketogulonicigenium, Kineococcus, Kingella, Klebsiella, Kocuria, Koribacter, Kosmotoga, Kribbella, Ktedonobacter, Kytococcus, Labrenzia, Lactobacillus, Lactococcus, Laribacter, Lautropia, Lawsonia, Legionella, Leifsonia, Lentisphaera, Leptolyngbya, Leptospira, Leptothrix, Leptotrichia, Leuconostoc, Liberibacter, Limnobacter, Listeria, Loktanella, Lutiella, Lyngbya, Lysinibacillus, Macrococcus, Magnetococcus, Magnetospirillum, Mahella, Mannheimia, Maricaulis, Marinithermus, Marinobacter, Marinomonas, Mariprofundus, Maritimibacter, Marvinbryantia, Megasphaera, Meiothermus, Melissococcus, Mesorhizobium, Methylacidiphilum, Methylibium, Methylobacillus, Methylobacter, Methylobacterium, Methylococcus, Methylocystis, Methylomicrobium, Methylophaga, Methylophilales, Methylosinus, Methyloversatilis, Methylovorus, Microbacterium, Micrococcus, Microcoleus, Microcystis, Microlunatus, Micromonospora, Mitsuokella, Mobiluncus, Moorella, Moraxella, Moritella, Mycobacterium, Myxococcus, Nakamurella, Natranaerobius, Neisseria, Neorickettsia, Neptuniibacter, Nitratifractor, Nitratiruptor, Nitrobacter, Nitrococcus, Nitrosomonas, Nitrosospira, Nitrospira, Nocardia, Nocardioides, Nocardiopsis, Nodularia, Nostoc, Novosphingobium, Oceanibulbus, Oceanicaulis, Oceanicola, Oceanithermus, Oceanobacillus, Ochrobactrum, Octadecabacter, Odyssella, Oligotropha, Olsenella, Opitutus, Oribacterium, Orientia, Ornithinibacillus, Oscillatoria, Oscillochloris, Oxalobacter, Paenibacillus, Pantoea, Paracoccus, Parascardovia, Parasutterella, Parvibaculum, Parvimonas, Parvularcula, Pasteurella, Pasteuria, Pectobacterium, Pediococcus, Pedosphaera, Pelagibaca, Pelagibacter, Pelobacter, Pelotomaculum, Peptoniphilus, Peptostreptococcus, Persephonella, Petrotoga, Phaeobacter, Phascolarctobacterium, Phenylobacterium, Photobacterium, Pirellula, Planctomyces, Planococcus, Plesiocystis, Polaromonas, Polaromonas, Polymorphum, Polynucleobacter, Poribacteria, Prochlorococcus, Propionibacterium, Proteus, Providencia, Pseudoalteromonas, Pseudoflavonifractor, Pseudomonas, Pseudonocardia, Pseudoramibacter, Pseudovibrio, Pseudoxanthomonas, Psychrobacter, Psychromonas, Puniceispirillum, Pusillimonas, Pyramidobacter, Rahnella, Ralstonia, Raphidiopsis, Regiella, Reinekea, Renibacterium, Rhizobium, Rhodobacter, Rhodococcus, Rhodoferax, Rhodomicrobium, Rhodopirellula, Rhodopseudomonas, Rhodospirillum, Rickettsia, Rickettsiella, Riesia, Roseburia, Roseibium, Roseiflexus, Roseobacter, Roseomonas, Roseovarius, Rothia, Rubrivivax, Rubrobacter, Ruegeria, Ruminococcus, Ruthia, Saccharomonospora, Saccharophagus, Saccharopolyspora, Sagittula, Salinispora, Salmonella, Sanguibacte, Scardovia, Sebaldella, Segniliparus, Selenomonas, Serratia, Shewanella, Shigella, Shuttleworthia, Sideroxydans, Silicibacter, Simonsiella, Sinorhizobium, Slackia, Sodalis, Solibacter, Solobacterium, Sorangium, Sphaerobacter, Sphingobium, Sphingomonas, Sphingopyxis, Spirochaeta, Sporosarcina, Stackebrandtia, Staphylococcus, Starkeya, Stenotrophomonas, Stigmatella, Streptobacillus, Streptococcus, Streptomyces, Streptosporangium, Subdoligranulum, subvibrioides, Succinatimonas, Sulfitobacter, Sulfobacillus, Sulfuricurvum, Sulfurihydrogenibium, Sulfurimonas, Sulfurospirillum, Sulfurovum, Sutterella, Symbiobacterium, Synechocystis, Syntrophobacter, Syntrophobotulus, Syntrophomonas, Syntrophothermus, Syntrophus, taiwanensis, Taylorella, Teredinibacter, Terriglobus, Thalassiobium, Thauera, Thermaerobacter, Thermanaerovibrio, Thermincola, Thermoanaerobacter, Thermoanaerobacterium, Thermobaculum, Thermobifida, Thermobispora, Thermocrinis, Thermodesulphateator, Thermodesulfobacterium, Thermodesulfobium, Thermodesulfovibrio, Thermomicrobium, Thermomonospora, Thermosediminibacter, Thermosinus, Thermosipho, Thermosynechococcus, Thermotoga, Thermovibrio, Thermus, Thioalkalimicrobium, Thioalkalivibrio, Thiobacillus, Thiomicrospira, Thiomonas, Tolumonas, Treponema, tribocorum, Trichodesmium, Tropheryma, Truepera, Tsukamurella, Turicibacter, Variovorax, Veillonella, Verminephrobacter, Verrucomicrobium, Verrucosispora, Vesicomyosocius, Vibrio, Vibrionales, Victivallis, Weissella, Wigglesworthia, Wolbachia, Wolinella, Xanthobacter, Xanthomonas, Xenorhabdus, Xylanimonas, Xylella, Yersinia, Zinderia and Zymomonas,*

In another example, the microbial cell may be a yeast cell. Suitable yeasts according to any aspect of the present invention may belong to the genera listed at http://www.dsmz.de/species/yeasts.htm. In particular, the yeast may be selected from the group consisting of *Schizosaccharomyces pombe, Schizosaccharomyces cerevisiae* and *Pichia pastoris.*

In particular, the microbial cell may be from selected from the group consisting of *Agrobacterium sp., Azotobacter sp., Bacillus sp, Enterobacter sp., Erwinia, sp., Escherichia sp., Klebsiella sp., Leucanea sp., Pantoea sp., Pectobacterium sp., Protaminobacter sp., Pseudomonas sp., Rauoltella sp. Salmonella sp.,* and *Serratia sp*.More in particular, the microbial cell may be *E. coli.*
The enzyme used according to any aspect of the present invention may be recombinant. The term "recombinant" as used herein, refers to a molecule or is encoded by such a molecule, particularly a polypeptide or nucleic acid that, as such, does not occur naturally but is the result of genetic engineering or refers to a cell that comprises a recombinant molecule. For example, a nucleic acid molecule is recombinant if it comprises a promoter functionally linked to a sequence encoding a catalytically active polypeptide and the promoter has been engineered such that the catalytically active polypeptide is overexpressed relative to the level of the polypeptide in the corresponding wild type cell that comprises the original unaltered nucleic acid molecule.

A skilled person would be able to use any method known in the art to genetically modify a cell or microorganism. According to any aspect of the present invention, the genetically modified cell may be genetically modified so that in a defined time interval, within 2 hours, in particular within 8 hours or 24 hours, it forms at least once or twice, especially at least 10 times, at least 100 times, at least 1000 times or at least 10000 times more isomaltulose than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (isomaltulose) in the nutrient medium. The genetically modified cell or microorganism may be genetically different from the wild type cell or microorganism. The genetic difference between the genetically modified microorganism according to any aspect of the present invention and the wild type microorganism may be in the presence of a point mutation in the genetically modified microorganism that may be absent in the wild type microorganism. In one example, the genetically modified microorganism according to any aspect of the present invention may comprise enzymes, in particular mutant SmuA that enables the microorganism to produce more isomaltulose and less by-products like trehalulose relative to the wild type cell with the wild type SmuA expression. The wild type microorganism relative to the genetically modified microorganism of the present invention may have none or no detectable activity of the enzymes. As used herein, the term 'genetically modified microorganism' may be used interchangeably with the term 'genetically modified cell'. The genetic modification according to any aspect of the present invention is carried out on the cell of the microorganism.

The cells according to any aspect of the present invention are genetically transformed according to any method known in the art. In particular, the cells may be produced according to the method disclosed in WO2013024114. The phrase 'the genetically modified cell has an increased activity, in comparison with its wild type, in enzymes' as used herein refers to the activity of the respective enzyme that is increased by a factor of at least 2, in particular of at least 10, more in particular of at least 100, yet more in particular of at least 1000 and even more in particular of at least 10000.

According to one aspect of the present invention, there is provided a cell that expresses a mutant sucrose isomerase enzyme according to any aspect of the present invention. According to yet another aspect of the present invention, there is provided a method of producing isomaltulose from a substrate comprising sucrose, wherein the method comprises:
- contacting the enzyme according to any aspect of the present invention with the substrate comprising sucrose in suitable conditions where sucrose is converted to isomaltulose or an isomaltulose composition.

According to a further aspect of the present invention, there is provided a method of producing isomaltulose from a substrate comprising sucrose, wherein the method comprises:
- contacting the cell according to any aspect of the present invention with the substrate comprising sucrose in suitable conditions where sucrose is converted to isomaltulose or an isomaltulose composition.

The term "substrate" means any substance or compound that is converted or meant to be converted into another compound by the action of an enzyme catalyst. This term according to any aspect of the present invention is a compound that comprises sucrose. In particular, substrate refers to sucrose-containing, aqueous solutions, for example aqueous solutions of sugar from sugar beet or sugar cane.

The reaction preferably takes place in an aqueous solution that is the substrate may be an aqueous solution so that the sucrose solution can contain water. Preferably the sucrose solution therefore contains 20 wt.% to 80 wt.%, preferably 30 wt.% to 70 wt.%, especially preferably 40 wt.% to 60 wt.% of water relative to the total sucrose.

The pH of the aqueous solution is preferably in the neutral or acid range, corresponding to a pH below 8.

The term "contacting", as used herein, means bringing about direct contact between the substrate and/or the cell according to any aspect of the present invention in an aqueous solution. For example, the cell and the substrate may not be in different compartments separated by a barrier such as an inorganic membrane. If the sugar is soluble and may be taken up by the cell or can diffuse across biological membranes, it may simply be added to the cell according to any aspect of the present invention in an aqueous solution. In case it is insufficiently soluble, it may be dissolved in a suitable organic solvent prior to addition to the aqueous solution.

In particular, the sucrose may be converted to an isomaltulose composition according to any aspect of the present invention and the composition comprises at least about 90, 91, 92, 93, 94, 95, 96, 97, 98 or 99% isomaltulose. In one example, the isomaltulose composition according to any aspect of the present invention comprises at least 20:1, 25:1, 30:1, 35:1, 40:1, 45:1, 50:1 isomaltulose to trehalulose ratio.

As used herein, "about" or "approximately" shall mean within 20 percent, particularly within 10 percent, and more particularly within 5 percent of a given value or range.

According to one aspect of the present invention, there is provided a use of the cell according to any aspect of the present invention, for producing isomaltulose from a substrate comprising sucrose.

### BRIEF DESCRIPTION OF THE FIGURES

Figure 1 is a graph characterizing SmuA(Y219L/D398G) via size exclusion chromatography and electrospray ionization mass spectrometry.
Figure 2 is an analysis graph of the product composition obtained by isomerization of sucrose by SmuA, SmuA(Y219L), SmuA(Y219L/D398G), SmuA(Q181P/D398G/F453Y) and SmuA(Y219L/T369F/D398G/F453Y).
Figure 3 is a graph showing the Michaelis-Menten kinetics of SmuA(Y219L/D398G) and HPLC chromatogram of the enzyme activity assay of SmuA.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Cloning and site-directed mutagenesis of the sucrose isomerase SmuA and its variants

The plasmid pASK-IBA4(+)-SmuA (SEQ ID NO:4) contains a synthetic gene, whose encoded amino acid sequence corresponds to the one deposited in the UniProt data bank (accession number D0VX20, SEQ ID NO:5), except for the single point mutation V465E and here used as wildtype SmuA. SmuA variants were generated by site-directed mutagenesis according to the QuikChange mutagenesis protocol (Agilent, Waldbronn, Germany) with suitable primers. Resulting expression plasmids coding for SmuA or its variants carrying an N-terminal *Strep*-tag II and an N-terminal OmpA signal sequence were confirmed by restriction analysis with *Kas*I and *Hind*III (New England Biolabs, Ipswich, MA) and DNA sequencing (Eurofins Genomics, Ebersberg, Germany).

### Example 2

### Periplasmic expression of SmuA and its variants in Escherichia coli and enzyme purification

*E. coli* KS272 (Strauch et al., 1988, Proc Natl Acad Sci USA 85, 1576-1580) transformed with one of the expression plasmids from Example 1 was used for expression of SmuA and its variants (Sambrook et al. (2001) Molecular cloning: a laboratory manual, 3rd edition. Cold Spring Harbor Laboratory Press). The primer sequences used are provided as SEQ ID NOs:6-115. Bacteria were grown at 37 °C to OD₅₅₀ = 0.5 in 2 L LB medium supplemented with 1 mM calcium acetate (Sigma-Aldrich, St. Louis, Missouri) and 100 µg/mL ampicillin (Carl-Roth, Karlsruhe, Germany) in a 5 L shake flask. SmuA production was induced upon addition of 0.2 µg/mL anhydrotetracycline (Acros Organics, Geel, Belgium) and cultivation was continued for 16 h at 16 °C.

Bacteria were harvested by centrifugation and resuspended in 20 mL 100 mM Tris/HCI pH 8.0, 0.5 M sucrose and 1 mM EDTA. After incubation on ice for 30 min, the suspension was centrifuged at 5,000 rpm for 10 min at 4 °C. The supernatant was clarified by a second centrifugation at 15,000 rpm for 15 min at 4 °C. The resulting periplasmic cell free extract was then dialyzed against SA buffer (100 mM Tris/HCI pH 8.0, 150 mM NaCl and 1 mM EDTA) over night at 4 °C.

The periplasmic protein extract was filtrated and applied on a *Strep*-Tactin Superflow column (IBA, Göttingen, Germany) with 2 mL bed volume, which had been pre-equilibrated with SA buffer. After washing with SA buffer, the bound protein was eluted with 2.5 mM desthiobiotin (IBA, Göttingen, Germany) in SA buffer. The elution fractions were concentrated via ultrafiltration (Amicon/Merck Millipore, Cork, Ireland) and purified via size exclusion chromatography. To this end, the sample solution was applied on a Superdex 200 10/300 GL column (GE Healthcare, Munich, Germany) using 20 mM Tris/HCI pH 8.0, 150 mM NaCl as running buffer. Several elution fractions were collected and further tested for purity via SDS-PAGE stained with Coomassie brilliant blue R-250 (Applichem GmbH, Darmstadt, Germany). Molecular mass was determined by electrospray ionization mass spectrometry on a maXis instrument (Bruker, Hamburg, Germany).

### Example 3

### Enzymatic assay and kinetic analysis of SmuA and its variants

The enzyme activity assay was carried out in 25 mM calcium acetate pH 5.5 in the presence of 1.16 M sucrose (Sigma-Aldrich, St. Louis, MO) for 16 h at 20 °C.

The reaction mixture was composed as follows:

| **Reagent or enzyme** | **Final concentration** |
|---|---|
| SmuA or variant | 74.7 nM |
| Calcium acetate pH 5.5 | 25 mM |
| Sucrose | 1.16 M |
| Total volume | 500 µL |

The enzymatic product composition was determined on an Agilent 1200 HPLC instrument (Agilent Technologies, Santa Clara, United States of America) equipped with a Smart line refractive index detector 2300 (Knauer, Berlin, Germany). The sample solution was applied on an XBridge amide column (particle size: 5.0 µm, inner diameter: 4.6, length: 250 mm); (Waters, Milford, MA) at a flow rate of 2 mL/min using a running buffer of 57.8 % acetonitrile, 26.1 % acetone and 16.1 % H₂O.

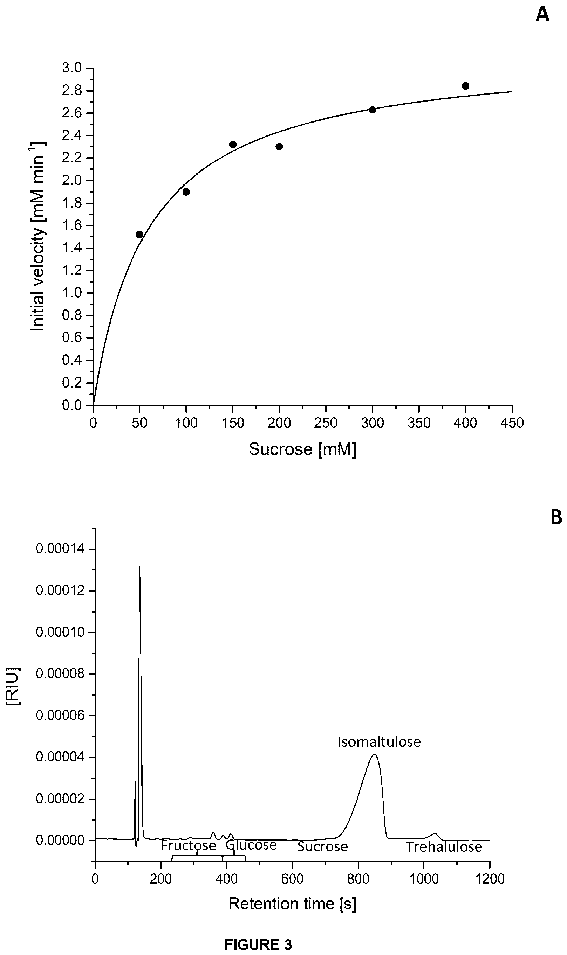

The Michaelis constant (K_{M}), the turnover number (k_{cat}) and the catalytic efficiency (k_{cat}/K_{M}) were determined for isomaltulose. To this end, the enzymatic reaction was carried out as described above at sucrose concentrations of 50 mM, 100 mM, 150 mM, 200 mM, 300 mM and 400 mM and stopped after 10 min, 20 min, 30 min and 40 min by heating the sample at 95 °C for 10 min. To determine the initial velocities at these sucrose concentrations, the isomaltulose yield was analysed via HPLC and plotted against the respective sucrose concentration. The slope of the subsequent linear regression corresponded to the initial velocity. The results are shown in Figure 3.

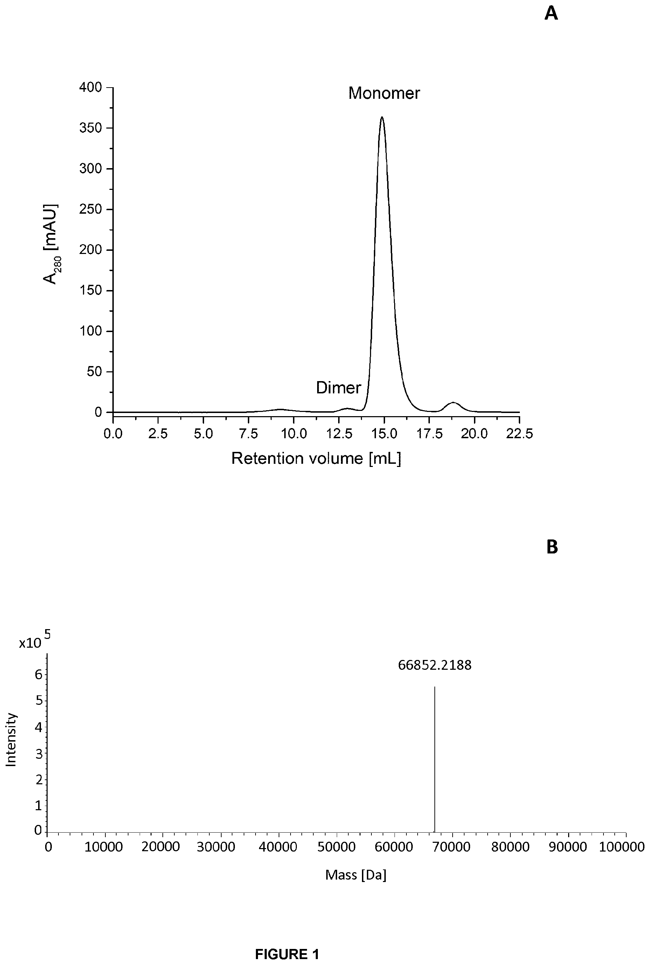

The analysis of various SmuA variants led to the detection of beneficial variants with strongly reduced yields of trehalulose (Tables 1 and 2). The results are also shown in Figures 1 to 3.

Figure 1 illustrates the size analysis of SmuA mutants. Panel (a) depicts the size exclusion chromatography of SmuA(Y219L/D398G). Chromatographic conditions: Superdex 200 10/300 GL; flow rate: 0.5 mL/min; injection volume: 500 µL; running buffer: 20 mM Tris/HCI pH 8.0, 150 mM NaCl. The elution profile was monitored by measuring the absorbance at 280 nm. An apparent size of 47.01 kDa was deduced with the help of the following protein calibration standards: apoferritin, alcohol deyhdrogenase, carboanhydrase, ovalbulmin and blue dextran (Sigma Aldrich, St. Louis, Missouri). Panel (b) depicts the electrospray ionization mass spectrometry of SmuA(Y219L/D398G). Protein sample: 40 µM dissolved in 10 mM ammonium acetate pH 6.6. Deconvoluted with MaxEntX (744.68-3004.90, *0.65625, 10000). Calculated molecular weight: 66,851.93 Da. Acquisition parameters: source type: ESI; focus: active; scan begin: 900 m/z; scan end: 3000 m/z; ion polarity: positive; set capillary: 3400 V; set end plate offset: -500 V; set charging voltage: 0 V; set corona: 0 nA; set nebulizer: 0.8 bar; set dry heater: 200 °C; set dry gas: 6.0 L/min; set divert valve: waste; set APCI heater: 0°C.

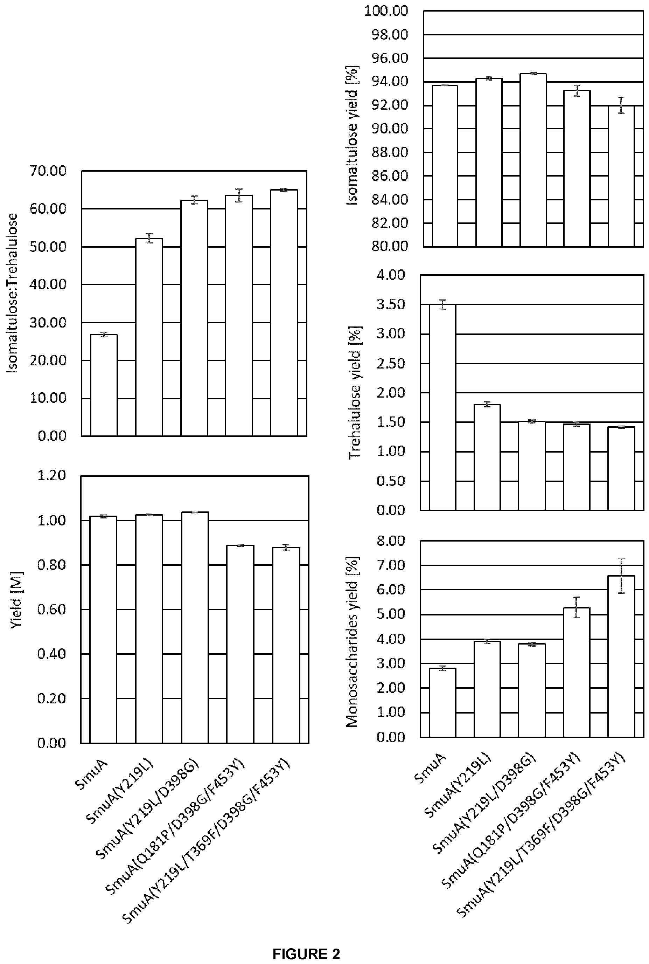

The bar diagrams in Figure 2 illustrate the ratio of isomaltulose to trehalulose and the percentage yield for isomaltulose, trehalulose and the monosaccharides. The enzyme activity assay was carried out in 500 µL of 25 mM calcium acetate buffer pH 5.5 containing 1.16 M sucrose for 16 h at 20 °C; the yield of isomaltulose is also depicted. The double mutant SmuA(Y219L/D398G) showed the highest yield of isomaltulose in this assay, both in absolute terms and as relative percentage of the product spectrum. While the mutants SmuA(Q181P/D398G/F453Y) and SmuA(Y219L/T369F/D398G/F453Y) exhibited progressively lower yields of the trehalulose side product, the yields for the main product isomaltulose were diminished and the proportion of monosaccharide side products was increased.

Figure 1 illustrates analysis of the enzyme kinetics and the product spectrum of mutant SmuA. Panel (a) shows the Michaelis-Menten kinetics for the production of isomaltulose by SmuA(Y219L/D398G). The initial velocities for the production of isomaltulose were measured by HPLC at sucrose concentrations of 50 mM, 100 mM, 150 mM, 200 mM, 300 mM, and 400 mM. The enzyme activity assay was carried out in 25 mM calcium acetate buffer pH 5.5 at 20 °C and 74.7 nM of the enzyme. Panel (b) shows the HPLC analysis of the enzyme activity assay with SmuA. Chromatographic conditions: XBridge amide column (particle size: 5.0 µm, inner diameter: 4.6, length: 250 mm); flow rate: 2 mL/min; injection volume: 20 µL; running buffer: 57.8 % acetonitrile, 26.1 % acetone, 16.1 % H₂O. Detection via Smartline refractive index detector 2300. The enzyme activity assay was carried out in 25 mM calcium acetate buffer pH 5.5 in the presence of 1.16 M sucrose and 74.4 nM of the recombinant wild type enzyme for 16 h at 20 °C.

**Table 1: Composition of the isomerization products from sucrose by engineered SmuA variants.**

| **Enzyme** | **Isomaltulose** | **Yield [%] Trehalulose** | **Monosaccharides** |
|---|---|---|---|
| SmuA | 93.70 ± 0.02 | 3.50 ± 0.08 | 2.80 ± 0.09 |
| SmuA(P67S) | 74.82 ± 0.61 | 1.30 ± 0.23 | 23.88 ± 0.75 |
| SmuA(Y78W) | 43.90 | 5.89 | 50.21 |
| SmuA(A179V) | 90.37 | 3.83 | 5.80 |
| SmuA(K180G) | 94.02 ± 0.07 | 3.58 ± 0.08 | 2.40 ± 0.04 |
| SmuA(K180V) | 93.72 ± 0.11 | 3.96 ± 0.05 | 2.32 ± 0.07 |
| SmuA(Q181P) | 94.12 ± 0.05 | 2.04 ± 0.05 | 3.84 ± 0.03 |
| SmuA(F213L) | 93.52 ± 0.13 | 3.57 ± 0.13 | 2.91 ± 0.02 |
| SmuA(V216A) | 94.14 ± 0.17 | 1.95 ± 0.09 | 3.92 ± 0.10 |
| SmuA(A217V) | 82.81 | 1.69 | 15.50 |
| SmuA(Y219L) | 94.70 ± 0.04 | 1.94 ± 0.02 | 3.36 ± 0.04 |
| SmuA(F236Y) | 92.86 ± 0.15 | 3.79 ± 0.15 | 3.35 ± 0.01 |
| SmuA(I269A) | 90.24 | 5.26 | 4.51 |
| SmuA(I269L) | 89.07 ± 0.33 | 8.01 ± 0.21 | 2.92 ± 0.13 |
| SmuA(I269V) | 94.62 ± 0.12 | 3.07 ± 0.09 | 2.31 ± 0.04 |
| SmuA(F270Y) | 95.25 ± 0.09 | 3.17 ± 0.03 | 1.58 ± 0.07 |
| SmuA(S277M) | 93.10 ± 0.05 | 4.37 ± 0.06 | 2.53 ± 0.05 |
| SmuA(S277Q) | 92.83 ± 0.14 | 2.43 ± 0.08 | 4.74 ± 0.07 |
| SmuA(S277T) | 91.67 ± 0.16 | 2.48 ± 0.08 | 5.84 ± 0.10 |
| SmuA(R284P) | 94.39 ± 0.23 | 2.21 ± 0.33 | 3.40 ± 0.23 |
| SmuA(F294Y) | 86.10 ± 0.08 | 0.88 ± 0.11 | 13.02 ± 0.06 |
| SmuA(I297G) | 72.55 | 4.12 | 23.34 |
| SmuA(L299G) | 93.14 | 3.85 | 3.01 |
| SmuA(D311P) | 93.21 ± 0.08 | 3.28 ± 0.04 | 3.51 ± 0.04 |
| SmuA(H341F) | 80.97 | 1.14 | 17.88 |
| SmuA(T369F) | 94.80 ± 0.09 | 2.30 ± 0.06 | 2.90 ± 0.11 |
| SmuA(T369W) | 93.83 ± 0.08 | 3.86 ± 0.07 | 2.31 ± 0.01 |
| SmuA(D398G) | 70.78 | 7.75 ± 3.72 | 21.47 ± 46.33 |
| SmuA(D398P) | 94.71 ± 0.18 | 2.71 ± 0.18 | 2.59 ± 0.04 |
| SmuA(D399E) | 72.23 | 4.74 | 23.03 |
| SmuA(D399N) | 93.02 ± 0.02 | 3.67 ± 0.05 | 3.31 ± 0.04 |
| SmuA(D399S) | 0.42 ± 8.60 | 0.42 ± 1.91 | 99.16 ± 10.51 |
| SmuA(I400A) | 77.82 | 5.38 | 16.80 |
| SmuA(I400V) | 92.51 | 3.88 | 3.61 |
| SmuA(I400Y) | 80.45 | 2.97 | 16.58 |
| SmuA(E401D) | 77.52 ± 0.92 | 2.34 ± 0.33 | 20.14 ± 0.94 |
| SmuA(E401Q) | 6.10 ± 0.18 | 7.85 ± 0.27 | 86.05 ± 0.23 |
| | | | |
| SmuA(L426V) | 94.82 ± 0.08 | 2.76 ± 0.12 | 2.42 ± 0.07 |
| SmuA(L426Y) | 94.68 ± 0.08 | 2.56 ± 0.08 | 2.76 ± 0.04 |
| SmuA(T427I) | 85.94 | 5.37 | 8.69 |
| SmuA(T427S) | 92.00 ± 0.10 | 3.84 ± 0.07 | 4.16 ± 0.03 |
| SmuA(T427V) | 86.96 | 4.50 | 8.54 |
| SmuA(S428A) | 91.14 | 4.39 | 4.47 |
| SmuA(S428T) | 90.94 | 3.92 | 5.14 |
| SmuA(D430A) | 81.14 | 3.94 | 14.92 |
| SmuA(D430E) | 87.12 | 3.51 | 9.37 |
| SmuA(D430L) | 79.08 | 3.75 | 17.17 |
| SmuA(D430N) | 84.35 ± 0.22 | 3.43 ± 0.12 | 12.22 ± 0.21 |
| SmuA(D430Q) | 81.96 | 3.66 | 14.38 |
| SmuA(D430S) | 86.33 ± 0.09 | 3.06 ± 0.09 | 10.62 ± 0.17 |
| SmuA(D430T) | 86.94 ± 0.10 | 2.94 ± 0.11 | 10.12 ± 0.20 |
| SmuA(N431Q) | 84.34 ± 0.53 | 13.84 ± 0.50 | 1.82 ± 0.04 |
| SmuA(N431S) | 87.26 ± 0.27 | 3.32 ± 0.09 | 9.42 ± 0.20 |
| SmuA(F453L) | 93.02 ± 0.23 | 3.69 ± 0.18 | 3.29 ± 0.07 |
| SmuA(F453W) | 90.96 ± 0.27 | 2.85 ± 0.21 | 6.19 ± 0.10 |
| SmuA(F453Y) | 94.59 ± 0.11 | 2.47 ± 0.15 | 2.94 ± 0.06 |
| SmuA(Y219L/D398G) | 94.69 ± 0.06 | 1.52 ± 0.02 | 3.79 ± 0.08 |
| SmuA(Q181P/D398G/F453Y) | 93.24 ± 0.44 | 1.47 ± 0.03 | 5.29 ± 0.41 |
| SmuA(Y219L/T369F/D398G/F453Y) | 92.00 ± 0.69 | 1.41 ± 0.02 | 6.59 ± 0.71 |

**Table 2: Kinetic parameters for the conversion of sucrose into isomaltulose by SmuA variants.**

| **Enzyme** | **K_{M} [mM]** | **k_{cat} [s⁻¹]** | **k_{cat}/K_{M} [mM⁻¹ s⁻¹]** |
|---|---|---|---|
| SmuA | 55.25 | 965.15 | 17.47 |
| SmuA(Y219L) | 86.64 | 665.77 | 7.68 |
| SmuA(Y219L/D398G) | 60.16 | 708.22 | 11.77 |
| SmuA(Q181P/D398G/F453Y) | 124.37 | 122.88 | 0.99 |
| SmuA(Y219L/T369F/D398G/F453Y) | 58.16 | 201.07 | 3.46 |

## Claims

1. An isolated sucrose isomerase enzyme (EC 5.4.99.11) comprising at least one point mutation in SEQ ID NO: 1, wherein the point mutation is selected from the group consisting of Q181P, V216A, Y219L, I269V, F270Y, S277Q, S277T, D311P, T369F, D398G, L426V, L426Y, F453Y and combinations thereof.

2. The enzyme according to claim 1, wherein the enzyme comprises a point mutation selected from the group consisting of Y219L, T369F, D398G, F453Y and combinations thereof.

3. The enzyme according to either claim 1 or 2, wherein the enzyme comprises a double mutation that comprises at least two point mutations selected from the group consisting of:
- D398G and F453Y,
- I269V and F453Y,
- F270Y and F453Y,
- Y219L and D398G,
- Q181P and I269V,
- Q181P and D398G,
- T369F and D398G and
- Y219L and F453Y.

4. The enzyme according to any one of the preceding claims, wherein the enzyme comprises a double mutation comprising the two point mutations Y219L and D398G.

5. The enzyme according to any one of the preceding claims, wherein the enzyme comprises a combination of point mutations selected from the group consisting of:
- Y219L and D398G,
- Q181P and I269V,
- Q181P and D398G,
- T369F and D398G and
- Y219L and F453Y.

6. The enzyme according to either claim 1 or 2, wherein the enzyme comprises a triple mutation that comprises at least three point mutations selected from the group consisting of:
- Q181P, D398G and F453Y,
- T369F, D398G and F453Y and
- T396F, D398G and L426Y.

7. The enzyme according to either claim 1 or 2, wherein the enzyme comprises a quadruple mutation that comprises at least four point mutations selected from the group consisting of:
- T396F, D398G, F453Y and Y219L,
- T369F, D398G, L426Y and I269V,
- T369F, D398G, L426Y and Y219L,
- T369F, D398G, L426Y and L426Y and
- T369F, D398G, L426Y and F453Y.

8. An isolated nucleic acid comprising a polynucleotide sequence that encodes the enzyme according to any one of claims 1 to 7.

9. An isolated cell comprising a vector comprising the nucleic acid of claim 8.

10. A microbial cell expressing a mutant sucrose isomerase enzyme, the mutant sucrose isomerase enzyme comprising at least one point mutation in SEQ ID NO: 1, wherein the point mutation is selected from the group consisting of Q181P, V216A, Y219L, I269V, F270Y, S277Q, S277T, D311P, T369F, D398G, L426V, L426Y, F453Y and combinations thereof.

11. The cell according to either claim 9 or 10, wherein the cell is selected from a group consisting of *Agrobacterium sp., Azotobacter sp., Bacillus sp, Enterobacter sp., Erwinia, sp., Escherichia sp., Klebsiella sp., Leucanea sp., Pantoea sp., Pectobacterium sp., Protaminobacter sp., Pseudomonas sp., Rauoltella sp. Salmonella sp.,* and *Serratia sp..*

12. The cell according to any one of claims 9 to 11, wherein the cell expresses a mutant sucrose isomerase enzyme according to any one of claims 1 to 7.

13. A method of producing isomaltulose from a substrate comprising sucrose, wherein the method comprises:
- contacting the enzyme according to any one of claims 1 to 7 with the substrate comprising sucrose in suitable conditions where sucrose is converted to isomaltulose or an isomaltulose composition; or
- contacting the cell according to any one of claims 9 to 12 with the substrate comprising sucrose in suitable conditions where sucrose is converted to isomaltulose or an isomaltulose composition.

14. The method according to claim 13, wherein the sucrose is converted to an isomaltulose composition and the composition comprises at least 90% isomaltulose and/or the composition comprises at least 20:1 isomaltulose to trehalulose ratio.

15. Use of the cell according to any one of claims 9 to 12, for producing isomaltulose from a substrate comprising sucrose.
